# EUROPÄISCHE PATENTSCHRIFT

(11) **EP 1 857 122 B1**
(45) Veröffentlichungstag und Bekanntmachung des Hinweises auf die Patenterteilung: **01.12.2010**
(21) Anmeldenummer: 07015835.7
(22) Anmeldetag: 05.06.2002
(51) Int. Cl.: C07K 14/11, C07K 14/47, A61K 48/00, A61K 39/00, A61K 39/145, A61P 31/16, A61P 35/04

(54) **Stabilisierte mRNA mit erhöhtem G/C-Gehalt, kodierend für ein virales Antigen**
Stabilised mRNA with increased G/C content, coding for a viral antigen
ARNm stabilisé avec un contenu augmenté en G/C, codant pour un antigène virale

(30) Priorität: 05.06.2001 DE 10127283
(43) Veröffentlichungstag der Anmeldung: 21.11.2007
(62) Teilanmeldung aus: 05004938.6
(73) Patentinhaber: CureVac GmbH, 72076 Tübingen (DE)
(72) Erfinder: von der Mülbe, Florian, 72070 Tübingen (DE); Pascolo, Steve, 72072 Tübingen (DE); Hoerr, Ingmar, 72070 Tübingen (DE)
(74) Vertreter: Graf von Stosch, Andreas

(56) Entgegenhaltungen:
- EP-A- 1 083 232
- WO-A-01/04313
- WO-A-02/08435
- WO-A-97/48370
- WO-A-98/34640
- WO-A-99/14346
- WO-A-99/20774
- WO-A-03/051401
- US-B1- 6 214 804
- LATHE R: "SYNTHETIC OLIGONUCLEOTIDE PROBES DEDUCED FROM AMINO ACID SEQUENCE DATA. THEORETICAL AND PRACTICAL CONSIDERATIONS" JOURNAL OF MOLECULAR BIOLOGY, LONDON, GB, Bd. 183, Nr. 1, 5. Mai 1985 (1985-05-05), Seiten 1-12, XP000674208 ISSN: 0022-2836
- HOERR INGMAR ET AL: "In vivo application of RNA leads to induction of specific cytotoxic T lymphocytes and antibodies" EUROPEAN JOURNAL OF IMMUNOLOGY, Bd. 30, Nr. 1, Januar 2000 (2000-01), Seiten 1-7, XP002243972 ISSN: 0014-2980
- HOLCIK M ET AL: "Four highly stable eukaryotic mRNAs assemble 3' untranslated region RNA-protein complexes sharing cis and trans components" PROCEEDINGS OF THE NATIONAL ACADEMY OF SCIENCES OF USA, NATIONAL ACADEMY OF SCIENCE, WASHINGTON, DC, US, Bd. 94, Nr. 6, 1997, Seiten 2410-2414, XP002243974 ISSN: 0027-8424
- DATABASE GENESEQ [Online] 20. Juli 1998 (1998-07-20), XP002466817 gefunden im EBI Database accession no. AAV21762 & WO 97/48370 A (MERCK & CO INC [US]; SHIVER JOHN W [US]; DAVIES MARY ELLEN [US]; FREED) 24. Dezember 1997 (1997-12-24)
- DATABASE CORENUCLEOTIDE [Online] 28. August 2002 (2002-08-28), XP002466818 gefunden im NCBI Database accession no. AF033819

### SEQUENCE LISTING

<110> CureVac GmbH
<120> Pharmazeutische Zusammensetzung, enthaltend eine stabilisierte und für die Translation in ihren codierenden Bereichen optimierte mRNA
<130> CU01P003WOEPT4
<150> PCT/EP02/06180
   <151> 2002-06-05
<160> 13
<170> PatentIn Ver. 2.1
<210> 1
   <211> 774
   <212> DNA
   <213> Influenza virus
<220>
   <223> Influenza-Matrix: Wildtyp-Gen (zum Vergleich)
<220>
   <223> Startcodon: atg (Nucleotide 11 bis 13), Stopcodon: tga (Nucleotide 767 bis 769)
<400> 1
   agatctaaag atgagtcttc taaccgaggt cgaaacgtac gttctctcta tcatcccgtc 60
   aggccccctc aaagccgaga tcgcacagag acttgaagat gtctttgcag ggaagaacac 120
   cgatcttgag gttctcatgg aatggctaaa gacaagacca atcctgtcac ctctgactaa 180
   ggggatttta ggatttgtgt tcacgctcac cgtgcccagt gagcgaggac tgcagcgtag 240
   acgctttgtc caaaatgccc ttaatgggaa cggggatcca aataacatgg acaaagcagt 300
   taaactgtat aggaagctca agagggagat aacattccat ggggccaaag aaatctcact 360
   cagttattct gctggtgcac ttgccagttg tatgggcctc atatacaaca ggatgggggc 420
   tgtgaccact gaagtggcat ttggcctggt atgtgcaacc tgtgaacaga ttgctgactc 480
   ccagcatcgg tctcataggc aaatggtgac aacaaccaac ccactaatca gacatgagaa 540
   cagaatggtt ttagccagca ctacagctaa ggctatggag caaatggctg gatcgagtga 600
   gcaagcagca gaggccatgg aggttgctag tcaggctagg caaatggtgc aagcgatgag 660
   aaccattggg actcatccta gctccagtgc tggtctgaaa aatgatcttc ttgaaaattt 720
   gcaggcctat cagaaacgaa tgggggtgca gatgcaacgg ttcaagtgaa ctag       774
<210> 2
   <211> 252
   <212> PRT
   <213> Influenza virus
<400> 2
   Met Ser Leu Leu Thr Glu Val Glu Thr Tyr Val Leu Ser Ile Ile Pro
    1               5                  10                  15
   Ser Gly Pro Leu Lys Ala Glu Ile Ala Gln Arg Leu Glu Asp Val Phe
               20                  25                  30
   Ala Gly Lys Asn Thr Asp Leu Glu Val Leu Met Glu Trp Leu Lys Thr
           35                  40                  45
   Arg Pro Ile Leu Ser Pro Leu Thr Lys Gly Ile Leu Gly Phe Val Phe
       50                  55                  60
   Thr Leu Thr Val Pro Ser Glu Arg Gly Leu Gln Arg Arg Arg Phe Val
   65                  70                  75                  80
   Gln Asn Ala Leu Asn Gly Asn Gly Asp Pro Asn Asn Met Asp Lys Ala
                   85                  90                  95
   Val Lys Leu Tyr Arg Lys Leu Lys Arg Glu Ile Thr Phe His Gly Ala
               100                 105                 110
   Lys Glu Ile Ser Leu Ser Tyr Ser Ala Gly Ala Leu Ala Ser Cys Met
           115                 120                 125
   Gly Leu Ile Tyr Asn Arg Met Gly Ala Val Thr Thr Glu Val Ala Phe
       130                 135                 140
   Gly Leu Val Cys Ala Thr Cys Glu Gln Ile Ala Asp Ser Gln His Arg
   145                 150                 155                 160
   Ser His Arg Gln Met Val Thr Thr Thr Asn Pro Leu Ile Arg His Glu
                   165                 170                 175
   Asn Arg Met Val Leu Ala Ser Thr Thr Ala Lys Ala Met Glu Gln Met
               180                 185                 190
   Ala Gly Ser Ser Glu Gln Ala Ala Glu Ala Met Glu Val Ala Ser Gln
           195                 200                 205
   Ala Arg Gln Met Val Gln Ala Met Arg Thr Ile Gly Thr His Pro Ser
       210                 215                 220
   Ser Ser Ala Gly Leu Lys Asn Asp Leu Leu Glu Asn Leu Gln Ala Tyr
   225                 230                 235                 240
   Gln Lys Arg Met Gly Val Gln Met Gln Arg Phe Lys
                   245                 250
<210> 3
   <211> 775
   <212> DNA
   <213> Artificial Sequence
<220>
   <223> Description of Artificial Sequence: Influenza-Matrix: Gen mit erhöhtem G/C-Gehalt
<220>
   <223> Startcodon: atg (Nucleotide 11 bis 13), Stopcodon: tga (Nucleotide 767 bis 769)
<400> 3
   agatctaaag atgagcctgc tgaccgaggt ggagacctac gtgctgagca tcatccccag 60
   cggccccctg aaggccgaga tcgcccagag gctggaggac gtgttcgccg gcaagaacac 120
   cgacctggag gtgctgatgg agtggctgaa gaccaggccc atcctgagcc ccctgaccaa 180
   gggcatcctg ggcttcgtgt tcaccctgac cgtgcccagc gagcgcggcc tgcagcgccg 240
   ccgcttcgtg cagaacgccc tgaacggcaa cggcgacccc aacaacatgg acaaggccgt 300
   gaagctgtac aggaagctga agagggagat caccttccac ggcgccaagg agatcagcct 360
   gagctacagc gccggcgccc tggccagctg catgggcctg atctacaaca ggatgggcgc 420
   cgtgaccacc gaggtggcct tcggcctggt gtgcgccacc tgcgagcaga tcgccgacag 480
   ccagcaccgc agccacaggc agatggtgac caccaccaac cccctgatca ggcacgagaa 540
   caggatggtg ctggccagca ccaccgccaa ggccatggag cagatggccg gcagcagcga 600
   gcaggccgcc gaggccatgg aggtggccag ccaggccagg cagatggtgc aggccatgag 660
   gaccatcggc acccacccca gcagcagcgc cggcctgaag aacgacctgc tggagaacct 720
   gcaggcctac cagaagcgca tgggcgtgca gatgcagcgc ttcaagtgaa ctagt      775
<210> 4
   <211> 844
   <212> DNA
   <213> Artificial Sequence
<220>
   <223> Description of Artificial Sequence: Influenza-Matrix: Gen für sekretierte Form (mit N-terminaler Signalsequenz) mit erhöhtem G/C-Gehalt
<220>
   <223> Startcodon: atg (Nucleotide 11 bis 13), Stopcodon: tga (Nucleotide 836 bis 838)
<400> 4
   agatctaaag atggccgtca tggccccccg caccctggtg ctgctgctga gcggcgccct 60
   ggccctgacc cagacctggg ctagcctgct gaccgaggtg gagacctacg tgctgagcat 120
   catccccagc ggccccctga aggccgagat cgcccagagg ctggaggacg tgttcgccgg 180
   caagaacacc gacctggagg tgctgatgga gtggctgaag accaggccca tcctgagccc 240
   cctgaccaag ggcatcctgg gcttcgtgtt caccctgacc gtgcccagcg agcgcggcct 300
   gcagcgccgc cgcttcgtgc agaacgccct gaacggcaac ggcgacccca acaacatgga 360
   caaggccgtg aagctgtaca ggaagctgaa gagggagatc accttccacg gcgccaagga 420
   gatcagcctg agctacagcg ccggcgccct ggccagctgc atgggcctga tctacaacag 480
   gatgggcgcc gtgaccaccg aggtggcctt cggcctggtg tgcgccacct gcgagcagat 540
   cgccgacagc cagcaccgca gccacaggca gatggtgacc accaccaacc ccctgatcag 600
   gcacgagaac aggatggtgc tggccagcac caccgccaag gccatggagc agatggccgg 660
   cagcagcgag caggccgccg aggccatgga ggtggccagc caggccaggc agatggtgca 720
   ggccatgagg accatcggca cccaccccag cagcagcgcc ggcctgaaga acgacctgct 780
   ggagaacctg caggcctacc agaagcgcat gggcgtgcag atgcagcgct tcaagtgaac 840
   tagt                                                              844
<210> 5
   <211> 942
   <212> RNA
   <213> Artificial Sequence
<220>
   <223> Description of Artificial Sequence: Influenza-Matrix: mRNA mit Stabilisierungssequenzen
<220>
<223> Die Stabilisierungssequenzen stammen aus den 5'- bzw. 3'-UTRs der β-Globin-mRNA von Xenopus laevis
<220>
   <223> Startcodon: aug (Nucleotide 56 bis 58), Stopcodon: uga (Nucleotide 812 bis 814)
<400> 5
   gcuuguucuu uuugcagaag cucagaauaa acgcucaacu uuggcagauc uaaagaugag 60
   ucuucuaacc gaggucgaaa cguacguucu cucuaucauc ccgucaggcc cccucaaagc 120
   cgagaucgca cagagacuug aagaugucuu ugcagggaag aacaccgauc uugagguucu 180
   cauggaaugg cuaaagacaa gaccaauccu gucaccucug acuaagggga uuuuaggauu 240
   uguguucacg cucaccgugc ccagugagcg aggacugcag cguagacgcu uuguccaaaa 300
   ugcccuuaau gggaacgggg auccaaauaa cauggacaaa gcaguuaaac uguauaggaa 360
   gcucaagagg gagauaacau uccauggggc caaagaaauc ucacucaguu auucugcugg 420
   ugcacuugcc aguuguaugg gccucauaua caacaggaug ggggcuguga ccacugaagu 480
   ggcauuuggc cugguaugug caaccuguga acagauugcu gacucccagc aucggucuca 540
   uaggcaaaug gugacaacaa ccaacccacu aaucagacau gagaacagaa ugguuuuagc 600
   cagcacuaca gcuaaggcua uggagcaaau ggcuggaucg agugagcaag cagcagaggc 660
   cauggagguu gcuagucagg cuaggcaaau ggugcaagcg augagaacca uugggacuca 720
   uccuagcucc agugcugguc ugaaaaauga ucuucuugaa aauuugcagg ccuaucagaa 780
   acgaaugggg gugcagaugc aacgguucaa gugaacuagu gacugacuag cccgcugggc 840
   cucccaacgg gcccuccucc ccuccuugca ccaaaaaaaa aaaaaaaaaa aaaaaaaaaa 900
   aaaaaaaaaa aaaaaaaaaa aaaaaaaaaa aaaaaaaaaa aa                    942
<210> 6
   <211> 942
   <212> RNA
   <213> Artificial Sequence
<220>
   <223> Description of Artificial Sequence: Influenza-Matrix: mRNA mit erhöhtem G/C-Gehalt und Stabilisierungssequenzen
<220>
   <223> Die Stabilisierungssequenzen stammen aus den 5'- bzw. 3'-UTRs der β-Globin-mRNA von Xenopus laevis
<220>
   <223> Startcodon: aug (Nucleotide 56 bis 58), Stopcodon: uga (Nucleotide 812 bis 814)
<400> 6
   gcuuguucuu uuugcagaag cucagaauaa acgcucaacu uuggcagauc uaaagaugag 60
   ccugcugacc gagguggaga ccuacgugcu gagcaucauc cccagcggcc cccugaaggc 120
   cgagaucgcc cagaggcugg aggacguguu cgccggcaag aacaccgacc uggaggugcu 180
   gauggagugg cugaagacca ggcccauccu gagcccccug accaagggca uccugggcuu 240
   cguguucacc cugaccgugc ccagcgagcg cggccugcag cgccgccgcu ucgugcagaa 300
   cgcccugaac ggcaacggcg accccaacaa cauggacaag gccgugaagc uguacaggaa 360
   gcugaagagg gagaucaccu uccacggcgc caaggagauc agccugagcu acagcgccgg 420
   cgcccuggcc agcugcaugg gccugaucua caacaggaug ggcgccguga ccaccgaggu 480
   ggccuucggc cuggugugcg ccaccugcga gcagaucgcc gacagccagc accgcagcca 540
   caggcagaug gugaccacca ccaacccccu gaucaggcac gagaacagga uggugcuggc 600
   cagcaccacc gccaaggcca uggagcagau ggccggcagc agcgagcagg ccgccgaggc 660
   cauggaggug gccagccagg ccaggcagau ggugcaggcc augaggacca ucggcaccca 720
   ccccagcagc agcgccggcc ugaagaacga ccugcuggag aaccugcagg ccuaccagaa 780
   gcgcaugggc gugcagaugc agcgcuucaa gugaacuagu gacugacuag cccgcugggc 840
   cucccaacgg gcccuccucc ccuccuugca ccaaaaaaaa aaaaaaaaaa aaaaaaaaaa 900
   aaaaaaaaaa aaaaaaaaaa aaaaaaaaaa aaaaaaaaaa aa                    942
<210> 7
   <211> 1011
   <212> RNA
   <213> Artificial Sequence
<220>
   <223> Description of Artificial Sequence: Influenza-Matrix: für sekretierte Form codierende mRNA mit erhöhtem G/C-Gehalt und Stabilisierungssequenzen
<220>
   <223> Startcodon: aug (Nucleotide 56 bis 58), Stopcodon: uga (Nucleotide 881 bis 883)
<400> 7
   gcuuguucuu uuugcagaag cucagaauaa acgcucaacu uuggcagauc uaaagauggc 60
   cgucauggcc ccccgcaccc uggugcugcu gcugagcggc gcccuggccc ugacccagac 120
   cugggccagc cugcugaccg agguggagac cuacgugcug agcaucaucc ccagcggccc 180
   ccugaaggcc gagaucgccc agaggcugga ggacguguuc gccggcaaga acaccgaccu 240
   ggaggugcug auggaguggc ugaagaccag gcccauccug agcccccuga ccaagggcau 300
   ccugggcuuc guguucaccc ugaccgugcc cagcgagcgc ggccugcagc gccgccgcuu 360
   cgugcagaac gcccugaacg gcaacggcga ccccaacaac auggacaagg ccgugaagcu 420
   guacaggaag cugaagaggg agaucaccuu ccacggcgcc aaggagauca gccugagcua 480
   cagcgccggc gcccuggcca gcugcauggg ccugaucuac aacaggaugg gcgccgugac 540
   caccgaggug gccuucggcc uggugugcgc caccugcgag cagaucgccg acagccagca 600
   ccgcagccac aggcagaugg ugaccaccac caacccccug aucaggcacg agaacaggau 660
   ggugcuggcc agcaccaccg ccaaggccau ggagcagaug gccggcagca gcgagcaggc 720
   cgccgaggcc auggaggugg ccagccaggc caggcagaug gugcaggcca ugaggaccau 780
   cggcacccac cccagcagca gcgccggccu gaagaacgac cugcuggaga accugcaggc 840
   cuaccagaag cgcaugggcg ugcagaugca gcgcuucaag ugaacuagug acugacuagc 900
   ccgcugggcc ucccaacggg cccuccuccc cuccuugcac caaaaaaaaa aaaaaaaaaa 960
   aaaaaaaaaa aaaaaaaaaa aaaaaaaaaa aaaaaaaaaa aaaaaaaaaa a 1011
<210> 8
   <211> 940
   <212> DNA
   <213> Homo sapiens
<220>
   <223> MAGE1: Wildtyp-Gen (zum Vergleich)
<220>
   <223> Startcodon: atg (Nucleotide 5 bis 7), Stopcodon: tga (Nucleotide 932 bis 934)
<400> 8
   catcatgtct cttgagcaga ggagtctgca ctgcaagcct gaggaagccc ttgaggccca 60
   acaagaggcc ctgggcctgg tgtgtgtgca ggctgccacc tcctcctcct ctcctctggt 120
   cctgggcacc ctggaggagg tgcccactgc tgggtcaaca gatcctcccc agagtcctca 180
   gggagcctcc gcctttccca ctaccatcaa cttcactcga cagaggcaac ccagtgaggg 240
   ttccagcagc cgtgaagagg aggggccaag cacctcttgt atcctggagt ccttgttccg 300
   agcagtaatc actaagaagg tggctgattt ggttggtttt ctgctcctca aatatcgagc 360
   cagggagcca gtcacaaagg cagaaatgct ggagagtgtc atcaaaaatt acaagcactg 420
   ttttcctgag atcttcggca aagcctctga gtccttgcag ctggtctttg gcattgacgt 480
   gaaggaagca gaccccaccg gccactccta tgtccttgtc acctgcctag gtctctccta 540
   tgatggcctg ctgggtgata atcagatcat gcccaagaca ggcttcctga taattgtcct 600
   ggtcatgatt gcaatggagg gcggccatgc tcctgaggag gaaatctggg aggagctgag 660
   tgtgatggag gtgtatgatg ggagggagca cagtgcctat ggggagccca ggaagctgct 720
   cacccaagat ttggtgcagg aaaagtacct ggagtaccgg caggtgccgg acagtgatcc 780
   cgcacgctat gagttcctgt ggggtccaag ggccctcgct gaaaccagct atgtgaaagt 840
   ccttgagtat gtgatcaagg tcagtgcaag agttcgcttt ttcttcccat ccctgcgtga 900
   agcagctttg agagaggagg aagagggagt ctgagcatga 940
<210> 9
   <211> 308
   <212> PRT
   <213> Homo sapiens
<220>
   <223> Tumorantigen MAGE1: Proteinsequenz
<400> 9
   Ser Leu Glu Gln Arg Ser Leu His Cys Lys Pro Glu Glu Ala Leu Glu
   1               5                   10                  15
   Ala Gln Gln Glu Ala Leu Gly Leu Val Cys Val Gln Ala Ala Thr Ser
               20                  25                  30
   Ser Ser Ser Pro Leu Val Leu Gly Thr Leu Glu Glu Val Pro Thr Ala
           35                  40                  45
   Gly Ser Thr Asp Pro Pro Gln Ser Pro Gln Gly Ala Ser Ala Phe Pro
       50                  55                  60
   Thr Thr Ile Asn Phe Thr Arg Gln Arg Gln Pro Ser Glu Gly Ser Ser
   65                  70                  75                  80
   Ser Arg Glu Glu Glu Gly Pro Ser Thr Ser Cys Ile Leu Glu Ser Leu
                   85                  90                  95
   Phe Arg Ala Val Ile Thr Lys Lys Val Ala Asp Leu Val Gly Phe Leu
               100                 105                 110
   Leu Leu Lys Tyr Arg Ala Arg Glu Pro Val Thr Lys Ala Glu Met Leu
           115                 120                 125
   Glu Ser Val Ile Lys Asn Tyr Lys His Cys Phe Pro Glu Ile Phe Gly
       130                 135                 140
   Lys Ala Ser Glu Ser Leu Gln Leu Val Phe Gly Ile Asp Val Lys Glu
   145                 150                 155                 160
   Ala Asp Pro Thr Gly His Ser Tyr Val Leu Val Thr Cys Leu Gly Leu
                   165                 170                 175
   Ser Tyr Asp Gly Leu Leu Gly Asp Asn Gln Ile Met Pro Lys Thr Gly
               180                 185                 190
   Phe Leu Ile Ile Val Leu Val Met Ile Ala Met Glu Gly Gly His Ala
           195                 200                 205
   Pro Glu Glu Glu Ile Trp Glu Glu Leu Ser Val Met Glu Val Tyr Asp
       210                 215                 220
   Gly Arg Glu His Ser Ala Tyr Gly Glu Pro Arg Lys Leu Leu Thr Gln
   225                 230                 235                 240
   Asp Leu Val Gln Glu Lys Tyr Leu Glu Tyr Arg Gln Val Pro Asp Ser
                   245                 250                 255
   Asp Pro Ala Arg Tyr Glu Phe Leu Trp Gly Pro Arg Ala Leu Ala Glu
               260                 265                 270
   Thr Ser Tyr Val Lys Val Leu Glu Tyr Val Ile Lys Val Ser Ala Arg
           275                 280                 285
   Val Arg Phe Phe Phe Pro Ser Leu Arg Glu Ala Ala Leu Arg Glu Glu
       290                 295                 300
   Glu Glu Gly Val
   305
<210> 10
   <211> 939
   <212> RNA
   <213> Artificial Sequence
<220>
   <223> Description of Artificial Sequence: MAGE1: mRNA mit erhöhtem G/C-Gehalt
<220>
   <223> Startcodon: aug (Nucleotide 1 bis 3), Stopcodon: uga (Nucleotide 937 bis 939)
<400> 10
   augagccugg agcagcgcag ccugcacugc aagccggagg aggcgcugga ggcgcagcag 60
   gaggcgcugg gccuggucug cguccaggcg gcgacgagca gcagcagccc gcugguccug 120
   ggcacgcugg aggagguccc gacggcgggc agcacggacc cgccgcagag cccgcagggc 180
   gcgagcgcgu ucccgacgac gaucaacuuc acgcgccagc gccagccgag cgagggcagc 240
   agcagccgcg aggaggaggg cccgagcacg agcugcaucc uggagagccu guuccgcgcg 300
   gucaucacga agaaggucgc ggaccugguc ggcuuccugc ugcugaagua ccgcgcgcgc 360
   gagccgguca cgaaggcgga gaugcuggag agcgucauca agaacuacaa gcacugcuuc 420
   ccggagaucu ucggcaaggc gagcgagagc cugcagcugg ucuucggcau cgacgucaag 480
   gaggcggacc cgacgggcca cagcuacguc cuggucacgu gccugggccu gagcuacgac 540
   ggccugcugg gcgacaacca gaucaugccg aagacgggcu uccugaucau cguccugguc 600
   augaucgcga uggagggcgg ccacgcgccg gaggaggaga ucugggagga gcugagcguc 660
   auggaggucu acgacggccg cgagcacagc gcguacggcg agccgcgcaa gcugcugacg 720
   caggaccugg uccaggagaa guaccuggag uaccgccagg ucccggacag cgacccggcg 780
   cgcuacgagu uccugugggg cccgcgcgcg cuggcggaga cgagcuacgu caagguccug 840
   gaguacguca ucaaggucag cgcgcgcguc cgcuucuucu ucccgagccu gcgcgaggcg 900
   gcgcugcgcg aggaggagga gggcgucuga gcgugauga                        939
<210> 11
   <211> 939
   <212> RNA
   <213> Artificial Sequence
<220>
   <223> Description of Artificial Sequence: MAGE1: mRNA mit alternativer Codonverwendung
<220>
<223> Startcodon: aug (Nucleotide 1 bis 3), Stopcodon: uga (Nucleotide 937 bis 939)
<400> 11
   augagccugg agcagcgcag ccugcacugc aagcccgagg aggcccugga ggcccagcag 60
   gaggcccugg gccuggugug cgugcaggcc gccaccagca gcagcagccc ccuggugcug 120
   ggcacccugg aggaggugcc caccgccggc agcaccgacc ccccccagag cccccagggc 180
   gccagcgccu uccccaccac caucaacuuc acccgccagc gccagcccag cgagggcagc 240
   agcagccgcg aggaggaggg ccccagcacc agcugcaucc uggagagccu guuccgcgcc 300
   gugaucacca agaagguggc  cgaccuggug ggcuuccugc ugcugaagua ccgcgcccgc 360
   gagcccguga ccaaggccga gaugcuggag agcgugauca agaacuacaa gcacugcuuc 420
   cccgagaucu ucggcaaggc cagcgagagc cugcagcugg uguucggcau cgacgugaag 480
   gaggccgacc ccaccggcca cagcuacgug cuggugaccu gccugggccu gagcuacgac 540
   ggccugcugg gcgacaacca gaucaugccc aagaccggcu uccugaucau cgugcuggug 600
   augaucgcca uggagggcgg ccacgccccc gaggaggaga ucugggagga gcugagcgug 660
   auggaggugu acgacggccg cgagcacagc gccuacggcg agccccgcaa gcugcugacc 720
   caggaccugg ugcaggagaa guaccuggag uaccgccagg ugcccgacag cgaccccgcc 780
   cgcuacgagu uccugugggg cccccgcgcc cuggccgaga ccagcuacgu gaaggugcug 840
   gaguacguga ucaaggugag cgcccgcgug cgcuucuucu uccccagccu gcgcgaggcc 900
   gcccugcgcg aggaggagga gggcguguga gccugauga                        939
<210> 12
   <211> 7
   <212> RNA
   <213> Artificial Sequence
<220>
   <223> Description of Artificial Sequence: Ein für eine Endonuklease erkennbares Sequenzmotiv, das im 3'UTR-Segment des für den Transferin-Rezeptor codierenden Gens enthalten ist (S. 10 der Beschreibung).
<400> 12
   gaacaag 7
<210> 13
   <211> 13
   <212> RNA
   <213> Artificial Sequence
<220>
   <223> Description of Artificial Sequence: Kozak-Sequenz, Ribosomen-Bindungsstelle (S. 12 der Beschreibung)
<400> 13
   gccgccacca ugg 13

## Beschreibung

Die vorliegende Erfindung betrifft eine pharmazeutische Zusammensetzung, enthaltend eine mRNA, die durch Sequenzmodifikationen im translatierten Bereich stabilisiert und für die Translation optimiert ist. Die erfindungsgemäße pharmazeutische Zusammensetzung eignet sich insbesondere als Impfstoff gegen virale Infektionskrankheiten. Des weiteren wird ein Verfahren zur Ermittlung von Sequenzmodifikationen, die der Stabilisierung und Translationsoptimierung von mRNA dienen, offenbart.

Die Gentherapie und die genetische Vakzinierung sind molekularmedizinische Verfahren, deren Anwendung in der Therapie und Prävention von Erkrankungen erhebliche Auswirkungen auf die medizinische Praxis haben wird. Beide Verfahren beruhen auf der Einbringung von Nukleinsäuren in Zellen bzw. in Gewebe des Patienten sowie auf der anschließenden Verarbeitung der durch die eingebrachten Nukleinsäuren codierten Information, d.h. der Expression der erwünschten Polypeptide.

Die übliche Vorgehensweise bisheriger Verfahren der Gentherapie und der genetischen Vakzinierung ist die Verwendung von DNA, um die benötigte genetische Information in die Zelle einzuschleusen. In diesem Zusammenhang sind verschiedene Verfahren zur Einbringung von DNA in Zellen, wie bspw. Calciumphosphat-Transfektion, Polypren-Transfektion, Protoplasten-Fusion, Elektroporation, Mikroinjektion und Lipofektion, entwickelt worden, wobei sich insbesondere die Lipofektion als geeignetes Verfahren herausgestellt hat.

Ein weiteres Verfahren, das insbesondere bei genetischen Vakzinierungsverfahren vorgeschlagen wurde, ist die Verwendung von DNA-Viren als DNA-Vehikel. Derartige Viren haben den Vorteil, daß aufgrund ihrer infektiösen Eigenschaften eine sehr hohe Transfektionsrate zu erzielen ist. Die verwendeten Viren werden genetisch verändert, so daß in der transfizierten Zelle keine funktionsfähigen infektiösen Partikel gebildet werden. Trotz dieser Vorsichtsmaßnahme kann jedoch aufgrund möglicher Rekombinationsereignisse ein gewisses Risiko der unkontrollierten Ausbreitung der eingebrachten gentherapeutisch wirksamen sowie viralen Gene nicht ausgeschlossen werden.

Üblicherweise wird die in die Zelle eingebrachte DNA in gewissem Ausmaß in das Genom der transfizierten Zelle integriert. Einerseits kann dieses Phänomen einen erwünschten Effekt ausüben, da hierdurch eine langandauernde Wirkung der eingebrachten DNA erzielt werden kann. Andererseits bringt die Integration in das Genom ein wesentliches Risiko der Gentherapie mit sich. So kann es bspw. zu einer Insertion der eingebrachten DNA in ein intaktes Gen kommen, was eine Mutation darstellt, welche die Funktion des endogenen Gens behindert oder gar vollkommen ausschaltet. Durch solche Integrationsereignisse können einerseits für die Zelle lebenswichtige Enzymsysteme ausgeschaltet werden, andererseits besteht auch die Gefahr einer Transformation der so veränderten Zelle in einen entarteten Zustand, falls durch die Integration der Fremd-DNA ein für die Regulation des Zellwachstums entscheidendes Gen verändert wird. Daher kann bei der Verwendung von DNA-Viren als Gentherapeutika und Vakzine ein Risiko der Krebsbildung nicht ausgeschlossen werden. In diesem Zusammenhang ist auch zu beachten, daß zur wirksamen Expression der in die Zelle eingebrachten Gene die entsprechenden DNA-Vehikel einen starken Promotor, bspw. den viralen CMV-Promotor, enthalten. Die Integration derartiger Promotoren in das Genom der behandelten Zelle kann zu unerwünschten Veränderungen der Regulierung der Genexpression in der Zelle führen.

Ein weiterer Nachteil der Verwendung von DNA als Gentherapeutika und Vakzine ist die Induktion pathogener Anti-DNA-Antikörper im Patienten unter Hervorrufung einer möglicherweise tödlichen Immunantwort.

Im Gegensatz zu DNA ist der Einsatz von RNA als Gentherapeutikum oder Vakzin als wesentlich sicherer einzustufen. Insbesondere bringt RNA nicht die Gefahr mit sich, stabil in das Genom der transfizierten Zelle integriert zu werden. Des weiteren sind keine viralen Sequenzen, wie Promotoren, zur wirksamen Transkription, erforderlich. Darüber hinaus wird RNA wesentlich einfacher *in vivo* abgebaut. Wohl aufgrund der gegenüber DNA relativ kurzen Halbwertszeit von RNA im Blutkreislauf sind bisher keine anti-RNA-Antikörper nachgewiesen worden. Daher kann RNA für molekularmedizinische Therapieverfahren als Molekül der Wahl angesehen werden.

Allerdings bedürfen auf RNA-Expressionssystemen beruhende medizinische Verfahren vor einer breiteren Anwendung noch der Lösung einiger grundsätzlicher Probleme. Eines der Probleme bei der Verwendung, von RNA ist der sichere, Zell- bzw. Gewebespezifische effiziente Transfer der Nukleinsäure. Da sich RNA in Lösung normalerweise als sehr instabil erweist, konnte durch die herkömmlichen Verfahren, die bei DNA verwendet werden, RNA bisher nicht oder nur sehr ineffizient als Therapeutikum bzw. Impfstoff verwendet werden.

Für die Instabilität sind RNA-abbauende Enzyme, sog. RNAasen (Ribonucleasen), verantwortlich. Selbst kleinste Verunreinigungen von Ribonucleasen reichen aus, um RNA in Lösung vollständig abzubauen. Der natürliche Abbau von mRNA im Cytoplasma von Zellen ist sehr fein reguliert. Diesbezüglich sind mehrere Mechanismen bekannt. So ist für eine funktionale mRNA die endständige Struktur von entscheidender Bedeutung. Am 5'-Ende befindet sich die sogenannte "Cap-Struktur" (ein modifiziertes Guanosin-Nucleotid) und am 3'-Ende eine Abfolge von bis zu 200 Adenosin-Nucleotiden (der sog. Poly-A-Schwanz). Über diese Strukturen wird die RNA als mRNA erkannt und der Abbau reguliert. Darüber hinaus gibt es weitere Prozesse, die RNA stabilisieren bzw. destabilisieren. Viele diese Prozesse sind noch unbekannt, oftmals scheint jedoch eine Wechselwirkung zwischen der RNA und Proteinen dafür maßgeblich zu sein. Bspw. wurde kürzlich ein "mRNA-Surveillance-System" beschrieben (Hellerin und Parker, Annu. Rev. Genet. 1999, 33: 229 bis 260), bei dem durch bestimmte Feedback-Protein-Wechselwirkungen im Cytosol unvollständige oder Nonsense-mRNA erkannt und dem Abbau zugänglich gemacht wird, wobei ein Hauptteil dieser Prozesse durch Exonucleasen vollzogen wird.

Im Stand der Technik sind einige Maßnahmen vorgeschlagen worden, um die Stabilität von RNA zu erhöhen und dadurch ihren Einsatz als Gentherapeutikum bzw. RNA-Vakzine zu ermöglichen.

In EP-A-1083232 wird zur Lösung des vorstehend genannten Problems der Instabilität von RNA *ex vivo* ein Verfahren zur Einbringung von RNA, insbesondere mRNA, in Zellen und Organismen vorgeschlagen, bei welchem die RNA in Form eines Komplexes mit einem kationischen Peptid oder Protein vorliegt.

WO 99/14346 beschreibt weitere Verfahren zur Stabilisierung von mRNA. Insbesondere werden Modifizierungen der mRNA vorgeschlagen, welche die mRNA-Spezies gegenüber dem Abbau von RNasen stabilisieren. Derartige Modifikationen betreffen einerseits die Stabilisierung durch Sequenzmodifikationen, insbesondere Verminderung des C- und/oder U-Gehalts durch Baseneliminierung oder Basensubstitution. Andererseits werden chemische Modifikationen, insbesondere die Verwendung von Nucleotidanaloga, sowie 5'- und 3'- Blockierungsgruppen, eine erhöhte Länge des Poly-A-Schwanzes sowie die Komplexierung der mRNA mit stabilisierenden Mitteln und Kombinationen der genannten Maßnahmen vorgeschlagen.

In den US-Patenten US 5,580,859 und US 6,214,804 werden unter anderem im Rahmen der "transienten Gentherapie" (TGT) mRNA-Vakzine und -Therapeutika offenbart. Es werden verschiedene Maßnahmen zur Erhöhung der Translationseffizienz und der mRNA-Stabilität beschrieben, die sich vor allem auf die nicht-translatierten Sequenzbereiche beziehen.

Bieler und Wagner (in: Schleef (Hrsg.), Plasmids for Therapy and Vaccination, Kapitel 9, Seiten 147 bis 168, Wiley-VCH, Weinheim, 2001) berichten von der Verwendung synthetischer Gene im Zusammenhang mit gentherapeutischen Methoden unter Verwendung von DNA-Vakzinen und lentiveralen Vektoren. Es wird die Konstruktion eines synthetischen, von HIV-1 abgeleiteten *gag*-Gens beschrieben, bei welchem die Codons gegenüber der Wildtyp-Sequenz derart modifiziert wurden (alternative Codonverwendung, engl. "codon usage"), daß sie der Verwendung von Codons entsprach, die in hoch exprimierten Säugergenen zu finden ist. Dadurch wurde insbesondere der A/T-Gehalt gegenüber der Wildtyp-Sequenz vermindert. Die Autoren stellen insbesondere eine erhöhte Expressionsrate des synthetischen *gag*-Gens in transfizierten Zellen fest. Des weiteren wurde in Mäusen eine erhöhte Antikörperbildung gegen das *gag*-Protein bei mit dem synthetischen DNA-Konstrukt immunisierten Mäusen und auch eine verstärkte Cytokinfreisetzung *in vitro* bei transfizierten Milzzellen von Mäusen beobachtet. Schließlich konnte eine Induzierung einer cytotoxischen Immunantwort in mit dem *gag*-Expressionsplasmid immunisierten Mäusen festgestellt werden. Die Autoren dieses Artikels führen die verbesserten Eigenschaften ihres DNA-Vakzins im wesentlichen auf einen durch die optimierte Codonverwendung hervorgerufene Veränderung des nucleo-cytoplasmatischen Transports der vom DNA-Vakzin exprimierten mRNA zurück. Im Gegensatz dazu halten die Autoren die Auswirkung der veränderten Codonverwendung auf die Translationseffizienz für gering.

Der vorliegenden Erfindung liegt daher die Aufgabe zugrunde, ein neues System zur genetischen Vakzinierung bereitzustellen, das die mit den Eigenschaften von DNA--Vakzinen verbundenen Nachteile überwindet und die Wirksamkeit von auf RNA-Spezies basierenden Therapeutika erhöht.

Diese Aufgabe wird durch die in den Ansprüchen gekennzeichneten Ausführungsformen der vorliegenden Erfindung gelöst.

Insbesondere wird eine modifizierte mRNA sowie eine mindestens eine derartige modifizierte mRNA in Verbindung mit einem pharmazeutisch verträglichen Träger und/oder Vehikel enthaltende pharmazeutische Zusammensetzung bereitgestellt, wobei die modifizierte mRNA für mindestens ein antigenes virales Peptid oder Polypeptid codiert, wobei die Sequenz der mRNA, insbesondere im für das mindestens eine Peptid oder Polypeptid codierenden Bereich, gegenüber der Wildtyp-mRNA folgende Modifikationen aufweist, die entweder alternativ oder in Kombination vorliegen können.

Zum einen ist der G/C-Gehalt des für das Peptid oder Polypeptid codierenden Bereichs der modifizierten mRNA größer als der G/C-Gehalt des codierenden Bereichs der für das Peptid oder Polypeptid codierenden Wildtyp-mRNA, wobei die codierte Aminosäuresequenz gegenüber dem Wildtyp unverändert ist.

Diese Modifikation beruht auf der Tatsache, daß für eine effiziente Translation einer mRNA die Sequenzabfolge des zu translatierenden Bereichs der mRNA wesentlich ist. Dabei spielt die Zusammensetzung und die Abfolge der verschiedenen Nucleotide eine große Rolle. Insbesondere sind Sequenzen mit erhöhtem G(Guanosin)/C(Cytosin)-Gehalt stabiler als Sequenzen mit einem erhöhten A(Adenosin)/U(Uracil)-Gehalt. Daher werden erfindungsgemäß unter Beibehaltung der translatierten Aminosäureabfolge die Codons gegenüber der Wildtyp-mRNA derart variiert, daß sie vermehrt G/C-Nucleotide beinhalten. Da mehrere Codons für ein und dieselbe Aminosäure codieren (Degeneration des genetischen Codes), können die für die Stabilität günstigsten Codons ermittelt werden (alternative Codonverwendung, engl. "codon usage").

In Abhängigkeit von der durch die modifizierte mRNA zu codierenden Aminosäure sind unterschiedliche Möglichkeiten zur Modifikation der mRNA-Sequenz gegenüber der Wildtyp-Sequenz möglich. Im Fall von Aminosäuren, die durch Codons codiert werden, die ausschließlich G- oder C- Nucleotide enthalten, ist keine Modifikation des Codons erforderlich. So erfordern die Codons für Pro (CCC oder CCG), Arg (CGC oder CGG), Ala (GCC oder GCG) und Gly (GCC oder GGG) keine Veränderung, da kein A oder U vorhanden ist.

In folgenden Fällen werden die Codons, welche A-und/oder U-Nucleotide enthalten durch Substituieren anderer Codons, welche die gleichen Aminosäuren codieren, jedoch kein A und/oder U enthalten, verändert. Beispiele sind:
Die Codons für Pro können von CCU oder CCA zu CCC oder CCG verändert werden;
die Codons für Arg können von CGU oder CGA oder AGA oder AGG zu CGC oder CGG verändert werden;
die Codons für Ala können von GCU oder GCA zu GCC oder GCG verändert werden;
die Codons für Gly können von GGU oder GGA zu GGC oder GGG verändert werden.

In anderen Fällen können A bzw. U-Nucleotide zwar nicht aus den Codons eliminiert werden, es ist jedoch möglich, den A- und U-Gehalt durch Verwendung von Codons zu vermindern, die weniger A- und/oder U-Nucleotide enthalten. Zum Beispiel:
Die Codons für Phe können von UUU zu UUC verändert werden;
die Codons für Leu können von UUA, CUU oder CUA zu CUC oder CUG verändert werden;
die Codons für Ser können von UCU oder UCA oder AGU zu UCC, UCG oder AGC verändert werden;
das Codon für Tyr kann von UAU zu UAC verändert werden;
das Stop-Codon UAA kann zu UAG oder UGA verändert werden;
das Codon für Cys kann von UGU zu UGC verändert werden;
das Codon His kann von CAU zu CAC verändert werden;
das Codon für Gln kann von CAA zu CAG verändert werden;
die Codons für Ile können von AUU oder AUA zu AUC verändert werden;
die Codons für Thr können von ACU oder ACA zu ACC oder ACG verändert werden;
das Codon für Asn kann von AAU zu AAC verändert werden;
das Codon für Lys kann von AAA zu AAG verändert werden;
die Codons für Val können von GUU oder GUA zu GUC oder GUG verändert werden;
das Codon für Asp kann von GAU zu GAC verändert werden;
das Codon für Glu kann von GAA zu GAG verändert werden.

Im Falle der Codons für Met (AUG) und Trp (UGG) besteht hingegen keine Möglichkeit der Sequenzmodifikation.

Die vorstehend aufgeführten Substitutionen können selbstverständlich einzeln aber auch in allen möglichen Kombinationen zur Erhöhung des G/C-Gehalts der modifizierten mRNA gegenüber der ursprünglichen Sequenz verwendet werden. So können beispielsweise alle in der ursprünglichen (Wildtyp-) Sequenz auftretenden Codons für Thr zu ACC (oder ACG) verändert werden. Vorzugsweise werden jedoch Kombinationen der vorstehenden Substitutionsmöglichkeiten genutzt, z.B.:
Substitution aller in der ursprünglichen Sequenz für Thr codierenden Codons zu ACC (oder ACG) und Substitution aller ursprünglich für Ser codierenden Codons zu UCC ( oder UCG oder AGC);
Substitution aller in der ursprünglichen Sequenz für Ile codierenden Codons zu AUC und
Substitution aller ursprünglich für Lys codierenden Codons zu AAG und Substitution aller ursprünglich für Tyr codierenden Codons zu UAC;
Substitution aller in der ursprünglichen Sequenz für Val codierenden Codons zu GUC (oder GUG) und Substitution aller ursprünglich für Glu codierenden Codons zu GAG und Substitution aller ursprünglich für Ala codierenden Codons zu GCC (oder GCG) und Substitution aller ursprünglich für Arg codierenden Codons zu CGC (oder CGG);
Substitution aller in der ursprünglichen Sequenz für Val codierenden Codons zu GUC (oder GUG) und Substitution aller ursprünglich für Glu codierenden Codons zu GAG und Substitution aller ursprünglich für Ala codierenden Codons zu GCC (oder GCG) und Substitution aller ursprünglich für Gly codierenden Codons zu GGC (oder GGG) und Substituion aller ursprünglich für Asn codierenden Codons zu AAC;
Substitution aller in der ursprünglichen Sequenz für Val codierenden Codons zu GUC (oder GUG) und Substitution aller usprünglich für Phe codierenden Codons zu UUC und Substitution aller ursprünglich für Cys codierenden Codons zu UGC und Substitution aller ursprünglich für Leu codierenden Codons zu CUG (oder CUC) und Substitution aller ursprünglich für Gln codierenden Codons zu CAG und Substitution aller ursprünglich für Pro codierenden Codons zu CCC (oder CCG);
usw.

Vorzugsweise wird der G/C-Gehalt des für das Peptid bzw. Polypeptid codierenden Bereichs der modifizierten mRNA um mindestens 7%-Punkte, mehr bevorzugt um mindestens 15%-Punkte, besonders bevorzugt um mindestens 20%-Punkte gegenüber dem G/C-Gehalt des codierten Bereichs der für das Polypeptid codierenden Wildtyp-mRNA erhöht.

Besonders bevorzugt ist es in diesem Zusammenhang, den G/C-Gehalt der modifizierten mRNA, insbesondere im für das mindestens eine Peptid bzw. Polypeptid codierenden Bereich, im Vergleich zur Wildtyp-Sequenz maximal zu erhöhen.

Die weitere erfindungsgemäße Modifikation der in der vorliegenden Erfindung gekennzeichneten pharmazeutischen Zusammensetzung enthaltenen mRNA beruht auf der Erkenntnis, daß die Translationseffizienz auch durch eine unterschiedliche Häufigkeit im Vorkommen von tRNAs in Zellen bestimmt wird. Sind daher in einer RNA-Sequenz vermehrt sogenannte "seltene" Codons vorhanden, so wird die entsprechende mRNA deutlich schlechter translatiert als in dem Fall, wenn für relativ "häufige" tRNAs codierende Codons vorhanden sind.

Somit wird erfindungsgemäß in der modifizierten mRNA (welche in der pharmazeutischen Zusammensetzung enthalten ist), der für das Peptid bzw. Polypeptid codierende Bereich gegenüber dem entsprechenden Bereich der Wildtyp-mRNA derart verändert, daß mindestens ein Codon der Wildtyp-Sequenz, das für eine in der Zelle relativ seltene tRNA codiert, gegen ein Codon ausgetauscht, das für eine in der Zelle relativ häufige tRNA codiert, welche die gleiche Aminosäure trägt wie die relativ seltene tRNA.

Durch diese Modifikation werden die RNA-Sequenzen derart modifiziert, daß Codons eingefügt werden, für die häufig vorkommende tRNAs zur Verfügung stehen.

Welche tRNAs relativ häufig in der Zelle vorkommen und welche demgegenüber relativ selten sind, ist einem Fachmann bekannt; vgl. bspw. Akashi, Curr. Opin. Genet. Dev. 2001, 11(6): 660-666.

Durch diese Modifikation können erfindungsgemäß alle Codons der Wildtyp-Sequenz, die für eine in der Zelle relativ seltene tRNA codieren, jeweils gegen ein Codon ausgetauscht werden, das für eine in der Zelle relativ häufige tRNA codiert, welche jeweils die gleiche Aminosäure trägt wie die relativ seltene tRNA.

Erfindungsgemäß besonders bevorzugt ist es, den erfindungsgemäß in der modifizierten mRNA erhöhten, insbesondere maximalen, sequenziellen G/C-Anteil mit den "häufigen" Codons zu verknüpfen, ohne die Aminosäuresequenz des durch den codierenden Bereich der mRNA codierten Peptids bzw. Polypeptids (ein oder mehrere) zu verändern. Diese bevorzugte Ausführungsform stellt eine besonders effizient translatierte und stabilisierte mRNA bspw. für die erfindungsgemäße pharmazeutische Zusammensetzung bereit.

In den Sequenzen eukaryotischer mRNAs gibt es destabilisierende Sequenzelemente (DSE), an welche Signalproteine binden und den enzymatischen Abbau der mRNA *in vivo* regulieren. Daher werden zur weiteren Stabilisierung der in der erfindungsgemäßen pharmazeutischen Zusammensetzung enthaltenen modifizierten mRNA gegebenenfalls im für das mindestens eine Peptid bzw. Polypeptid codierenden Bereich ein oder mehrere Veränderungen gegenüber dem entsprechenden Bereich der Wildtyp-mRNA vorgenommen, so daß keine destabilisierenden Sequenzelemente enthalten sind. Selbstverständlich ist es erfindungsgemäß ebenfalls bevorzugt, gegebenenfalls in den nicht-translatierten Bereichen (3'- und/oder 5'-UTR) vorhandene DSE aus der mRNA zu eliminieren.

Derartige destabilisierende Sequenzen sind bspw. AU-reiche Sequenzen ("AURES"), die in 3'-UTR-Abschnitten zahlreicher instabiler mRNA vorkommen (Caput et al., Proc. Natl. Acad. Sci. USA 1986, 83: 1670 bis 1674). Die in der erfindungsgemäßen pharmazeutischen Zusammensetzung enthaltenen RNA-Moleküle sind daher vorzugsweise derart gegenüber der Wildtyp-mRNA verändert, daß sie keine derartigen destabilisierenden Sequenzen aufweisen. Dies gilt auch für solche Sequenzmotive, die von möglichen Endonucleasen erkannt werden, bspw. die Sequenz GAACAAG, die im 3' UTR-Segment des für den Transferin-Rezeptor codierenden Gens enthalten ist (Binder et al., EMBO J. 1994, 13: 1969 bis 1980). Auch diese Sequenzmotive werden bevorzugt in der modifizierten mRNA der erfindungsgemäßen pharmazeutischen Zusammensetzung eliminiert.

Einem Fachmann sind verschiedene Verfahren geläufig, die zur Substitution von Codons in der erfindungsgemäßen modifizierten mRNA geeignet sind. Im Falle kürzerer codierender Bereiche (die für biologisch wirksame oder antigene Peptide codieren) kann bspw. die gesamte mRNA chemisch unter Verwendung von Standardtechniken synthetisiert werden.

Bevorzugt werden allerdings Basensubstitutionen unter Verwendung einer DNA-Matrize zur Herstellung der modifizierten mRNA mit Hilfe von Techniken der gängigen zielgerichteten Mutagenese eingeführt; Maniatis et al., Molecular Cloning: A Laboratory Manual, Cold Spring Harbor Laboratory Press, 3. Aufl., Cold Spring Harbor, NY, 2001.

Bei diesem Verfahren wird zur Herstellung der mRNA daher ein entsprechendes DNA-Molekül *in vitro* transkribiert. Diese DNA-Matrize besitzt einen geeigneten Promotor, bspw. einen T7 -oder SP6-Promotor, für die *in vitro* Transkription, dem die gewünschte Nucleotidsequenz für die herzustellende mRNA und ein Termisationssignal für die in in vitro Transkribtion folgen. Erfingdungsgemäß wird das DNA-Molekül, das die Matrize des herzustellenden RNA-Konstrukts bildet, durch fermentative Vermehrung und anschlieβende Isolierung als Teil eines in Bakterien replizierbaren Plasmids hergestellt. Als für die vorliegende Erfindung geeignete Plasmide können bspw. die Plasmide pT7Ts (GenBank-Zugriffsnummer U26404; Lai et al., Development 1995, 121: 2349 bis 2360), pGEM^{®}-Reihe, bspw. pGEM^{®}-1 (GenBank-Zugrifsnummer X65300; von Promega) und pSP64 (GenBank-Zugriffsnummer X65327) genannt werden; vgl. auch Mezei und Storts, Purification of PCR Products, in: Griffin und Griffin (Hrsg.), PCR Technology: Current Innovation, CRC Press, Boca Raton, FL, 2001.

Es kann so unter Verwendung kurzer synthetischer DNA-Oligonucleotide, die an den entstehenden Schnittstellen kurze einzelsträngige Übergänge aufweisen, oder durch chemische Synthese hergestellte Gene die gewünschte Nucleotidsequenz nach einem Fachmann geläufigen molekularbiologischen Methoden in ein geeignetes Plasmid cloniert werden (vgl. Maniatis et al., s.o.). Das DNA-Molekül wird dann aus dem Plasmid, in welchem es in einfacher oder mehrfacher Kopie vorliegen kann, durch Verdauung mit Restriktionsendonukleasen ausgeschnitten.

Die modifizierte mRNA, welche in der erfindungsgemäßen pharmazeutischen Zusammensetzung enthalten ist, kann darüber hinaus eine 5'-Cap-Struktur (ein modifiziertes Guanosin-Nucleotid) aufweisen. Als Beispiele von Cap-Strukturen können m7G(5')ppp (5'(A,G(5')ppp(5')A und G(5')ppp(5')G genannt werden.

Gemäß einer weiteren bevorzugten Ausführungsform der vorliegenden Erfindung enthält die modifizierte mRNA einen PolyA-Schwanz von mindestens 50 Nuclotiden, vorzugsweise mindestens 70 Nuclotiden, mehr bevorzugt mindestens 100 Nuclotiden, besonders bevorzugt mindestens 200 Nuclotiden.

Für eine effiziente Translation der mRNA ist weiterhin eine wirksame Bindung der Ribosomen an die Ribosomen-Bindungsstelle (Kozak-Sequenz: GCCGCCACCAUGG, das AUG bildet das Startcodon) erforderlich. Diesbezüglich ist festgestellt worden, daß ein erhöhter A/U-Gehalt um diese Stelle herum eine effizientere Ribosomen-Bindung an die mRNA ermöglicht.

Des weiteren ist es möglich, in die modifizierte mRNA eine oder mehrere sog. IRES (engl. "internal ribosomal entry side) einzufügen. Eine IRES kann so als alleinige Ribosomen-Bindungsstelle fungieren, sie kann jedoch auch zur Bereitstellung einer mRNA dienen, die mehrere Peptide bzw. Polypeptide codiert, die unabhängig voneinander durch die Ribosomen translatiert werden sollen ("multicistronische mRNA"). Beispiele erfindungsgemäß verwendbarer IRES-Sequenzen sind diejenigen aus Picornaviren (z.B. FMDV), Pestviren (CFFV), Polioviren (PV), Enzephalo-Myocarditis-Viren (ECMV), Maul-und-Klauenseuche-Viren (FMDV), Hepatitis-C-Viren (HCV), Klassisches-Schweinefieber-Viren (CSFV), Murines-Leukoma-Virus (MLV), Simean-Immundefizienz-Viren (SIV) oder Cricket-Paralysis-Viren (CrPV).

Gemäß einer weiteren bevorzugten Ausführungsform der vorligenden Erfindung weist die modifizierte mRNA in den 5'- und/oder 3'-nicht translatierten Bereichen Stabilisierungssequenzen auf, die befähigt sind, die Halbwertszeit der mRNA im Cytosol zu erhöhen.

Diese Stabilisierungssequenzen können eine 100%ige Sequenzhomologie zu natürlich vorkommenden Sequenzen, die in Viren, Bakterien und Eukaryoten auftreten, aufweisen, können aber auch teilweise oder vollständig synthetischer Natur sein. Als Beispiel für stabilisierende Sequenzen, die in der vorliegenden Erfindung verwendbar sind, können die nicht-translatierten Sequenzen (UTR) des β-Globingens, bspw. von *Homo sapiens* oder *Xenopus laevis,* genannt werden. Ein anderes Beispiel einer Stabilisierungssequenz weist die allgemeine Formel (C/U)CCANₓCCC(U/A)PyₓUC(C/U)CC auf, die im 3'UTR der sehr stabilen mRNA enthalten ist, die für α-Globin, α-(I)-Collagen, 15-Lipoxygenase oder für Tyrosin-Hydroxylase codiert (vgl. Holcik et al., Proc. Natl. Acad. Sci. USA 1997,94: 2410 bis 2414). Selbstverständlich können derartige Stabilisierungssequenzen einzeln oder in Kombination miteinander als auch in Kombination mit anderen, einem Fachmann bekannten Stabilisierungssequenzen verwendet werden.

Zur weiteren Stabilisierung der modifizierten mRNA ist es außerdem bevorzugt, daß diese mindestens ein Analoges natürlich vorkommender Nucleotide aufweist. Dies beruht auf der Tatsache, daß die in den Zellen vorkommenden RNA-abbauenden Enzyme als Substrat vorzugsweise natürlich vorkommende Nucleotide erkennen. Durch Einfügen von Nucleotidanaloga kann daher der RNA-Abbau erschwert werden, wobei die Auswirkung auf die Translationseffizienz bei Einfügen von diesen Analoga, insbesondere in den codierenden Bereich der mRNA, einen positiven oder negativen Effekt auf die Translationseffizienz haben kann.

In einer keineswegs abschließenden Aufzählung können als Beispiele erfindungsgemäß verwendbarer Nucleotidanaloga Phosphoramidate, Phosphorthioate, Peptidnucleotide, Methylphosphonate, 7-Deazaguaonsin, 5-Methylcytosin und Inosin genannt werden. Die Herstellung derartiger Analoga sind einem Fachmann bspw. aus den US-Patenten 4,373,071, US 4,401,796, US 4,415,732, US 4,458,066, US 4,500,707, US 4,668,777, US 4,973,679, US 5,047,524, US 5,132,418, US 5,153,319, US 5,262,530 und 5,700,642 bekannt. Erfindungsgemäß können derartige Analoga in nicht-translatierten und translatierten Bereichen der modifizierten mRNA vorkommen.

Des weiteren kann der wirksame Transfer der modifizierten mRNA in die zu behandelnden Zellen bzw. den zu behandelnden Organismus dadurch verbessert werden, daß die modifizierte mRNA mit einem kationischen Peptid oder Protein assoziiert oder daran gebunden ist. Insbesondere ist dabei die Verwendung von Protamin als polykationischem, Nucleinsäure-bindenden Protein besonders wirksam. Des weiteren ist die Verwendung anderer kationischer Peptide oder Proteine, wie Poly-L-Lysin oder Histonen, ebenfalls möglich. Diese Vorgehensweise zur Stabilisierung der modifizierten mRNA ist in EP-A-1083232 beschrieben.

Ein weiterer Anwendungsbereich der vorliegenden Erfindung ist die Vakzinierung, d.h. die Verwendung der modifizierten mRNA zur Impfung bzw. die Verwendung der pharmazeutischen Zusamensetzung als Impfstoff bzw. die Verwendung der modifizierten mRNA zur Herstellung der pharmazeutischen Zusammesetzung zur Impfung. Die Vakzinierung beruht auf der Einbringung eines Viralen Antigens, im vorliegenden Fall der genetischen Information für das Antigen in Form der für das Antigen codierenden modifizierten mRNA, in den Organismus, insbesondere in die Zelle. Die in der pharmazeutischen Zusammensetzung enthaltene modifizierte mRNA wird in das Antigen translatiert, d.h. das von der modifizierten mRNA codierte Polypeptid bzw. antigene Peptid wird exprimiert, wodurch eine gegen dieses Polypeptid bzw. antigene Peptid gerichtete Immunantwort stimuliert wird. Bei der Vakzinierung gegen einen pathologischen Keim, d.h. ein Virus, wird daher ein Oberflächenantigen eines derartigen Keims zur Vakzinierung mit Hilfe der erfindungsgemäßen pharmazeutischen Zusammensetzung, enthaltend die für das Oberflächenantigen codierende modifizierte mRNA, verwendet.

Des weiteren wird die erfindungsgemäße pharmazeutische Zusammensetzung gegen virale Infektionskrankheiten, wie AIDS (HIV), Hepatitis A, B oder C, Herpes, Herpes Zoster (Varizellen), Röteln (Rubeola-Virus), Gelbfieber, Dengue usw. (Flavi-Viren), Grippe (Influenza-Viren), hämorrhagische Infektionskrankheiten (Marburg- oder Ebola-Viren eingesetzt. Vorzugsweise werden auch im Falle von Infektionseikrankungen die entsprechenden Oberflächenantigene mit dem stärksten antigenen Potenzial durch die modifizierte mRNA codiert. Bei den genannten Genen pathogener viraler Keime ist dies typischerweise eine sekretierte Form eines Oberflächenantigens. Des weiteren werden erfindungsgemäß bevorzugt für Polypeptide codierende mRNAs eingesetzt, wobei es sich bei den Polypeptiden um Polyepitope, bspw. der vorstehend genannten Antigene, insbesondere Oberflächenantigene von pathogenen viralen Keimen, vorzugsweise sekretierter Proteinformen, handelt.

Darüber hinaus kann die erfindungsgemäß modifizierte mRNA neben dem antigenen oder dem gentherapeutisch wirksamen Peptid bzw. Polypeptid auch mindestens einen weiteren funktionalen Abschnitt enthalten, der bspw. für ein die Immunantwort förderndes Cytokin (Monokin, Lymphokin, Interleukin oder Chemokin, wie IL-1, IL-2, IL-3, IL-4, IL-5, IL-6, IL-7, IL-8, IL-9, IL-10, IL-12, INF-α, INF-γ, GM-CFS, LT-α oder Wachstumsfaktoren, wie hGH, codiert.

Des weiteren kann zur Erhöhung der Immunogenizität die erfindungsgemäße pharmazeutische Zusammensetzung ein oder mehrere Adjuvanzien enthalten. Unter "Adjuvans" ist dabei jede chemische oder biologische Verbindung zu verstehen, die eine spezifische Immunantwort begünstigt. In Abhängigkeit der verschiedenen Arten von Adjuvanzien können diesbezüglich verschiedene Mechanismen in Betracht kommen. Bspw. bilden Verbindungen, die eine Endocytose der in der pharmazeutischen Zusammensetzung enthaltenen modifizierten mRNA durch dendritische Zellen (DC) fordern, eine erste Klasse von verwendbaren Adjuvanzien. Andere Verbindungen, welche die Reifung der DC erlauben, bspw. Lipopolysaccharide, TNF-a oder CD40-Ligand, sind eine weitere Klasse geeigneter Adjuvanzien. Allgemein kann jedes das Immunsystem beeinflussende Agens von der Art eines "Gefahrsignals" (LPS, GP96, Oligonucleotide mit dem CpG-Motiv) oder Cytokine, wie GM-CFS, als Adjuvans verwendet werden, welche es erlauben, eine Immunantwort gegen ein Antigen, das durch die modifizierte mRNA codiert wird, zu erhöhen und/oder gerichtet zu beeinflussen. Insbesondere sind dabei die vorstehend genannten Cytokine bevorzugt. Weitere bekannte Adjuvanzien sind Aluminiumhydroxid, das Freud'sche Adjuvans sowie die vorstehend genannten stabilisierenden kationischen Peptide bzw. Polypeptide, wie Protamin. Des weiteren sind Lipopeptide, wie Pam3Cys, ebenfalls besonders geeignet, um als Adjuvanzien in der pharmazeutischen Zusammmensetzung der vorliegenden Erfindung eingesetzt zu werden; vgl. Deres et al, Nature 1989, 342: 561-564.

Die erfindungsgemäße pharmazeutische Zusammensetzung enthält neben der modifizierten mRNA einen pharmazeutisch verträglichen Träger und/oder ein pharmazeutisch verträgliches Vehikel. Entsprechende Wege zur geeigneten Formulierung und Herstellung der erfindungsgemäßen pharmazeutischen Zusammensetzung sind bei "Remington's Pharmaceutical Sciences" (Mack Pub. Co., Easton, PA, 1980) offenbart, das vollinhaltlich Bestandteil der Offenbarung der vorliegenden Erfindung ist. Für die parenterale Verabreichung kommen als Trägerstoffe bspw. steriles Wasser, sterile Kochsalzlösungen, Polyalkylenglykole, hydrogenierte Naphthalen und insbesondere biokompatible Lactidpolymere, Lactid/Glycolidcopolymer oder Polyoxyethylen-/Polyoxypropylencopolymere in Betracht. Erfindungsgemäße Zusammensetzungen können Füllsubstanzen oder Substanzen, wie Lactose, Mannitol, Substanzen zur kovalenten Anknüpfung von Polymeren, wie z.B. Polyethylenglykol an erfindungsgemäße Inhibitoren, Komplexierung mit Metallionen oder Einschluß von Materialien in oder auf besondere Präparationen von Polymerverbindung, wie z.B. Polylactat, Polyglykolsäure, Hydrogel oder auf Liposomen, Mikroemulsion, Micellen, unilamellare oder multilamellare Vesikel, Erythrozyten-Fragmente oder Sphäroplasten, enthalten. Die jeweiligen Ausführungsformen der Zusammensetzungen werden abhängig vom physikalische Verhalten, beispielsweise in Hinblick auf die Löslichkeit, die Stabilität, Bioverfügbarkeit oder Abbaubarkeit gewählt. Kontrollierte oder konstante Freisetzung der erfindungsgemäßen Wirkstofikomponente in der Zusammensetzung schließt Formulierungen auf der Basis lipophiler Depots ein (z.B. Fettsäuren, Wachse oder Öle). Im Rahmen der vorliegenden Erfindung werden auch Beschichtungen erfindungsgemäßer Substanzen oder Zusammensetzungen, enthaltend solche Substanzen, nämlich Beschichtungen mit Polymeren offenbart (z.B. Poloxamere oder Poloxamine). Weiterhin können erfindungsgemäßen Substanzen bzw. Zusammensetzungen protektive Beschichtungen, z.B. Proteaseinhibitoren oder Permeabilitätsverstärker, aufweisen. Bevorzugte Träger sind typischerweise wässrige Trägermaterialien, wobei Wasser zur Injektion (WFI) oder Wasser, gepuffert mit Phosphat, Citrat oder Acetat usw. verwendet wird, und der pH typischerweise auf 5,0 bis 8,0, vorzugsweise 6,0 bis 7,0, eingestellt wird. Der Träger bzw. das Vehikel wird zusätzlich vorzugsweise Salzbestandteile enthalten, z.B. Natriumchlorid, Kaliumchlorid oder andere Komponenten, welche die Lösung bspw. isotonisch machen. Weiterhin kann der Träger bzw. das Vehikel neben den vorstehend genannten Bestandteilen zusätzliche Komponenten, wie humanes Serumalbumin (HSA), Polysorbat 80, Zucker oder Aminosäuren, enthalten.

Die Art und Weise der Verabreichung und die Dosierung der erfindungsgemäßen pharmazeutischen Zusammensetzung hängen von der zu behandelnden Erkrankung und deren Fortschrittsstadium, wie auch dem Körpergewicht, dem Alter und dem Geschlecht des Patienten ab.

Die Konzentration der modifizierten mRNA in derartigen Formulierungen kann daher innerhalb eines weiten Bereichs von 1 µg bis 100 mg/ml variieren. Die erfindungsgemäße pharmazeutische Zusammensetzung wird vorzugsweise parenteral, bspw. intravenös, intraarteriell, subkutan, intramuskulär, dem Patienten verabreicht. Ebenso ist es möglich, die pharmazeutische Zusammensetzung topisch oder oral zu verabreichen.

Somit wird erfindungsgemäß auch ein Verfahren zur Behandlung der vorstehend genannten Krankheiten bzw. ein Impfverfahren zur Prävention der vorstehend genannten Erkrankungen bereitgestellt, welches das Verabreichen der erfindungsgemäßen pharmazeutischen Zusammensetzung an einen Patienten, insbesondere einen Menschen, umfasst.

Darüber hinaus wird ebenfalls ein Verfahren bereitgestellt, dass der Ermittlung der modifizierten Sequenz der in der erfindugsgemäßen pharmazeutischen Zusammensetzung enthaltenen mRNA dient. Dabei wird erfindugsgemäß die Adaptierung der RNA-Sequenzen mit zwei unterschiedlichen Optimierungszielen durchgeführt: Zum einen mit größtmöglichen G/C-Gehalt, zum anderen unter bestmöglicher Berücksichtigung der Häufigkeit der tRNAs gemäß Codon Usage. Im ersten Schritt des Verfahrens wird eine virtuelle Translation einer beliebigen RNA(oder DNA)-Sequenz durchgeführt, um die entsprechende Aminosäuresequenz zu generieren. Ausgehend von der Aminosäuresequenz wird eine virtuelle reverse Translation durchgeführt, die aufgrund des degenerierten genetischen Codes Wahlmöglichkeiten für die entsprechenden Codons liefert. Je nach geforderter Optimierung bzw. Modifizierung werden zur Auswahl der geeigneten Codons entsprechende Selektionslisten und Optimierungsalgorithmen verwendet. Die Ausführung der Algorithmen erfolgt typischerweise mit Hilfe einer geeigneten Software auf einem Computer. So wird die optimierte mRNA-Sequenz erstellt und kann bspw. mit Hilfe einer entsprechenden Anzeigevorrichtung ausgegeben und mit der usrpünglichen (Wildtyp-)Sequenz verglichen werden. Das gleiche gilt auch für die Häufigkeit der einzelnen Nucleotide. Die Änderungen gegenüber der ursprünglichen Nucleotidsequenz werden dabei vorzugsweise hervorgehoben. Weiter werden gemäß einer bevorzugten Ausführungsform in der Natur bekannte stabile Sequenzen eingelesen, welche die Grundlage für eine gemäß natürlichen Sequenzmotiven stabilisierte RNA bieten können. Es kann ebenfalls eine Sekundärstrukturanalyse vorgesehen werden, die anhand von Strukturberechnungen stabilisierende und instabilisierende Eigenschaften bzw. Bereiche der RNA analysieren kann.

Die Figuren zeigen:

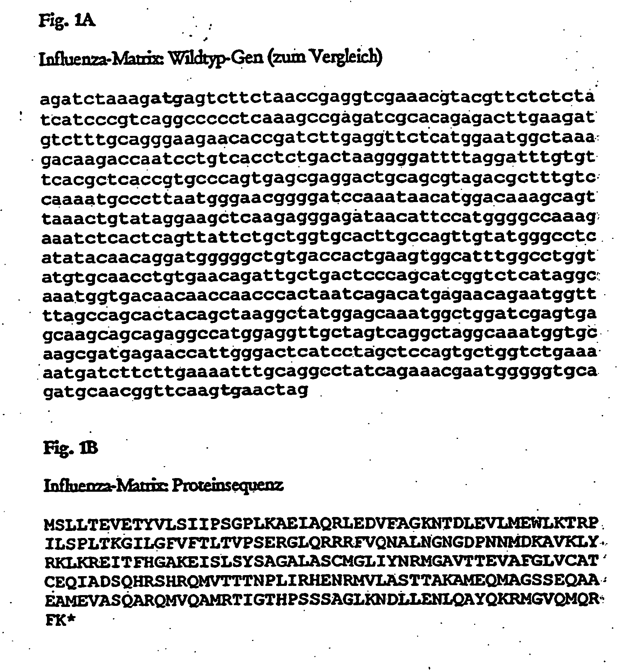

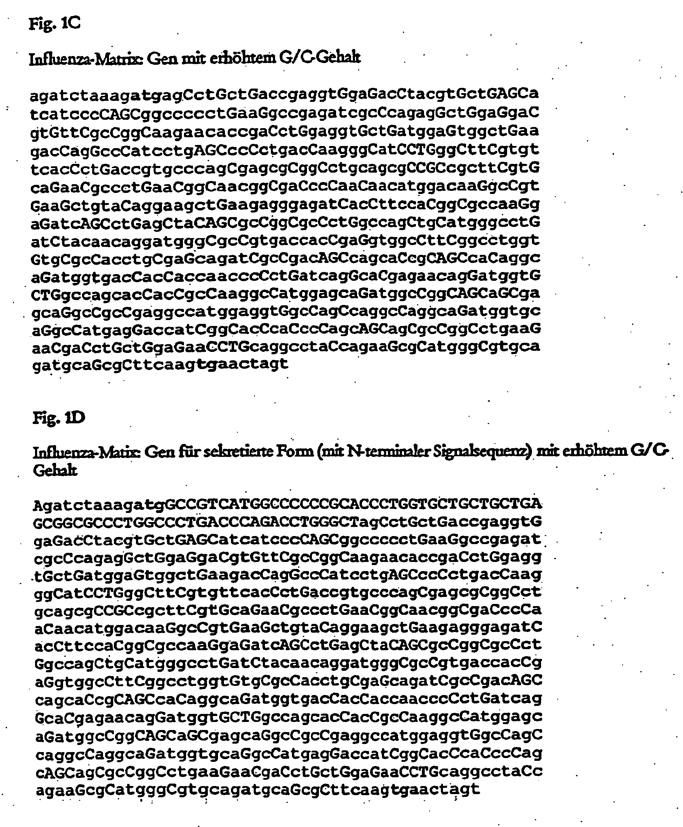

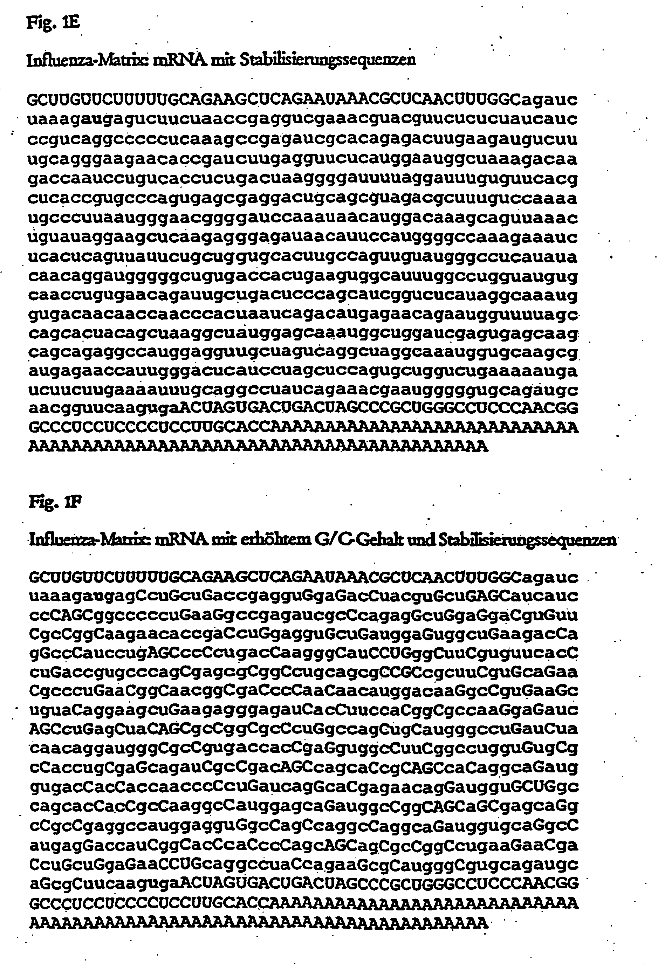

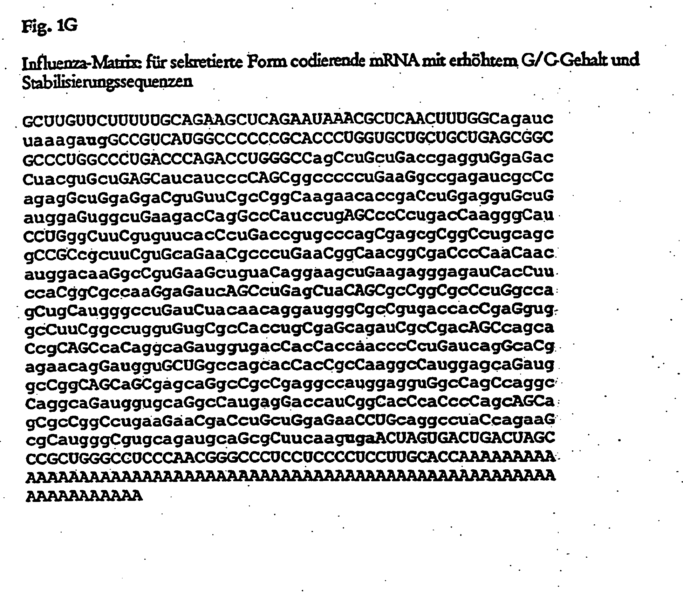

Fig. 1 zeigt Wildtyp- und modifizierte Sequenzen für das Influenza-Matrix-Protein.
Fig. 1A zeigt das Wildtyp-Gen und Fig. 1B zeigt die davon abgeleitete Aminosäuresequenz (Ein-Buchstaben-Code). Fig. 1C zeigt eine für das Influenza-Matrix-Protein codierende Gen-Sequenz, deren G/C-Gehalt gegenüber der Wildtyp-Sequenz erhöht ist. Fig. 1D zeigt die Sequenz eines Gens, das für eine sekretierte Form des Influenza-Matrix-Proteins codiert (einschließlich einer N-terminalen Signalsequenz), wobei der G/C-Gehalt der Sequenz gegenüber der Wildtyp-Sequenz erhöht ist. Fig. 1E zeigt eine für das Influenza-Matrix-Protein codierende mRNA, die im Vergleich zur Wildtyp-mRNA stabilisierende Sequenzen enthält. Fig. 1F zeigt eine für das Influenza-Matrix-Protein codierende mRNA, die neben stabilisierenden Sequenzen einen erhöhten G/C-Gehalt aufweist.
Fig. 1G zeigt ebenfalls eine modifizierte mRNA, die für die sekretierte Form des Influenza-Matrix-Proteins codiert und im Vergleich zum Wildtyp stabilisierende Sequenzen und einen erhöhten GC-Gehalt aufweist. In Fig. 1A und Fig. 1C bis 1G sind die Start- und Stop-Codons fett hervorgehoben. Gegenüber der Wildtyp-Sequenz von Fig. 1A veränderte Nucleotide sind in den Figuren 1C bis 1G in Großbuchstaben dargestellt.

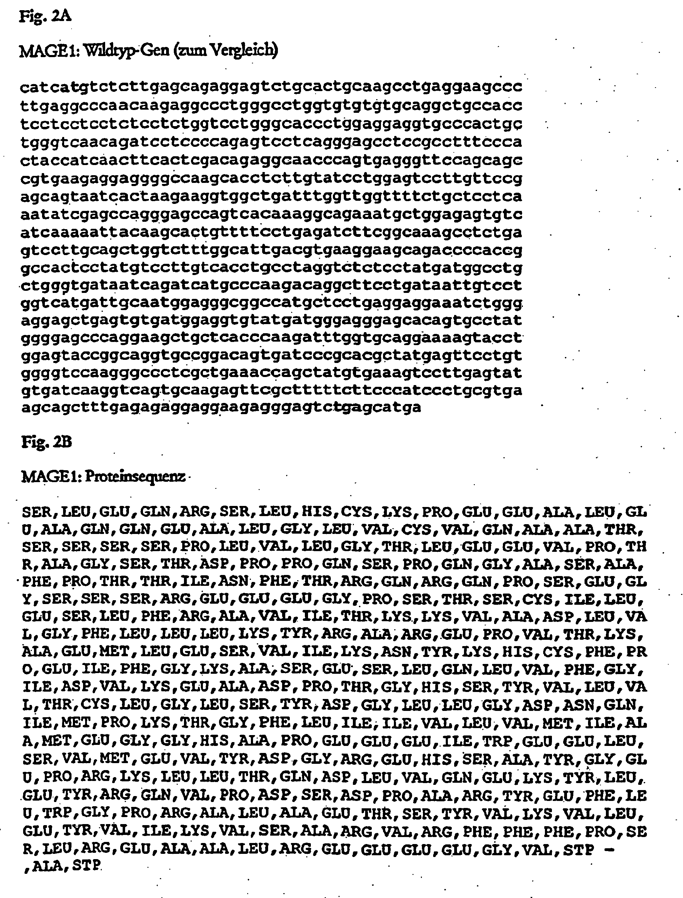

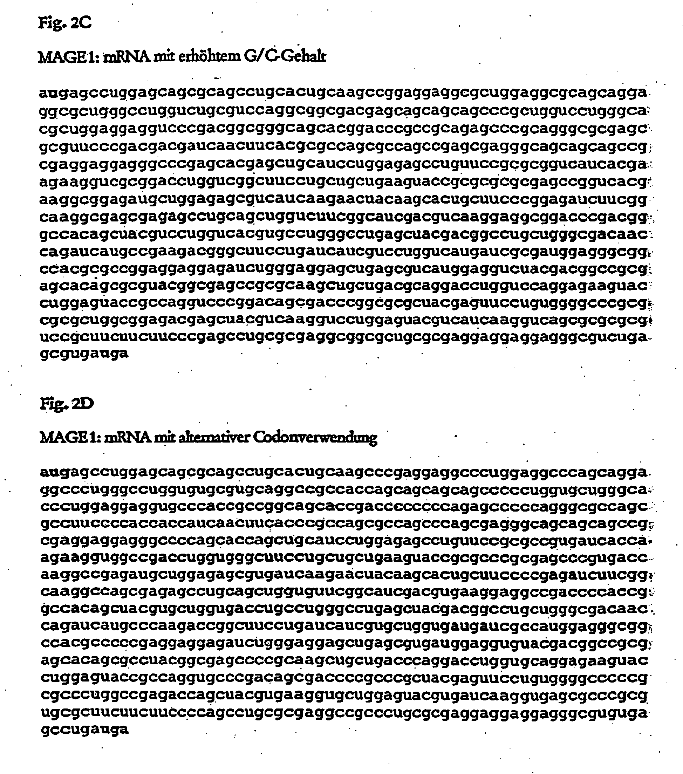

Fig. 2 zeigt Wildtyp- und erfindungsgemäß modifizierte Sequenzen, die für das Tumorantigen MAGE1 codieren.
Fig. 2A zeigt die Sequenz des Wildtyp-Gens und Fig. 2B die davon abgeleitete Aminosäuresequenz (Drei-Buchstaben-Code). Fig. 2C zeigt eine für MAGE1 codierende modifizierte mRNA, deren G/C-Gehalt gegenüber dem Wildtyp erhöht ist. Fig. 2D zeigt die Sequenz einer für MAGE1 codierenden modifizierten mRNA, bei der die Codon-Verwendung bezüglich der Codierung möglichst häufig in der Zelle vorkommender tRNA optimiert wurde. Start- und Stop-Codons sind jeweils fett gekennzeichnet.

Die folgenden Beispiele erläutern die vorliegende Erfindung näher, ohne sie einzuschränken.

### Beispiel 1

Als beispielhafte Ausführungsform des Verfahrens zu Ermittlung der Sequenz einer erfindungsgemäß modifizierten mRNA wurde ein Computerprogramm erstellt, das mit Hilfe des genetischen Codes bzw. dessen degenerativer Natur die Nucleotid-Sequenz einer beliebigen mRNA derart modifiziert, daß sich ein maximaler G/C-Gehalt in Verbindung mit der Verwendung von Codons, die für möglichst häufig in der Zelle vorkommende tRNAs codieren, ergibt, wobei die durch die modifizierte mRNA codierte Aminosäure-Sequenz gegenüber der nicht-modifizierten Sequenz identisch ist. Alternativ kann auch nur der G/C-Gehalt oder nur die Codonverwendung gegenüber der ursprünglichen Sequenz modifiziert werden.

Der Quellcode in Visual Basic 6.0 (eingesetzte Entwicklungsumgebung: Microsoft Visual Studio Enterprise 6.0 mit Servicepack 3) ist im Anhang angegeben.

### Beispiel 2

Mit Hilfe des Computerprogramms von Beispiel 1 wurde ein RNA-Konstrukt mit einer hinsichtlich Stabilisierung und Translationseffizienz optimierten Sequenz des lac-Z-Gens aus *E.coli* erstellt. Es konnte so ein G/C-Gehalt von 69% (im Vergleich zur Wildtyp-Sequenz von 51%; vgl. Kalnins et al., EMBO J. 1983, 2(4): 593-597) erzielt werden. Durch die Synthese überlappender Oligonucleotide, welche die modifizierte Sequenz aufweisen, wurde die optimierte Sequenz gemäß im Stand der Technik bekannter Verfahren hergestellt. Die endständigen Oligonucleotide wiesen die folgenden Restriktionsschnittstellen auf: Am 5'-Ende eine *Eco*RV- sowie am 3'-Ende eine *Bgl*II-Schnittstelle. Über Verdau mit *Eco*RV/*Bgl*II wurde die modifizierte lacZ-Sequenz in das Plasmid pT7Ts (GenBank-Zugriffsnummer U 26404; vgl. Lai et al., s.o.). pT7Ts enthält als nichttranslatierte Regionen Sequenzen aus dem β-Globingen von *Xenopus levis* jeweils an 5' und 3'. Das Plasmid wurde vor dem Einfügen der modifizierten lacZ-Sequenz mit den genannten Restriktionsenzymen geschnitten.

Das pT7Ts-lac-Z-Konstrukt wurde in Bakterien vermehrt und durch Phenol-Chloroform-Extraktion gereinigt. 2 µg des Konstrukts wurden *in vitro* mittels dem einem Fachmann bekannten Verfahren transkribiert, wodurch die modifizierte mRNA hergestellt wurde.

### Beispiel 3

Mit Hilfe des erfindungsgemäßen Computerprogramms von Beispiel 1 wurde das Gen für das Influenza-Matrix-Protein (Wildtyp-Sequenz vgl. Fig. 1A, abgeleitete Aminosäuresequenz Fig. 1 B) optimiert. Dadurch wurde die in Fig. 1C dargestellte G/C-reiche Sequenzvariante erstellt. Ebenfalls wurde eine für die sekretierte Form des Influenza-Matrix-Proteins codierende G/C-reiche Sequenz, die für eine N-terminale Signalsequenz codiert, ermittelt (vgl. Fig. 1D). Die sekretierte Form des Influenza-Matrix-Proteins hat den Vorteil, daß sie eine im Vergleich zur nicht-sekretierten Form erhöhte Immunogenizität aufweist.

Ausgehend von den optimierten Sequenzen wurden entsprechende mRNA-Moleküle entworfen. Die hinsichtlich G/C-Gehalt und Codonverwendung optimierte RNA für das Influenza-Matrix-Protein wurde zusätzlich mit stabilisierenden Sequenzen im 5'- und 3'-Bereich (die Stabilisierungssequenzen stammen aus den 5'- bzw. 3'-UTRs des β-Globin-mRNA von *Xenupus laevis;* vgl. pT7Ts-Vektor in Lai et al., s.o.) ausgestattet (vgl. Fig. 1E und 1F). Desgleichen wurde auch die für die sekretierte Form des Influenza-Matrix-Proteins codierende mRNA im translatierten Bereich sequenzoptimiert und mit den genannten stabilisierenden Sequenzen versehen (vgl. Fig. 1G).

### Vergleichs-Beispiel 4

Mit Hilfe des Computerprogramms von Beispiel 1 wurde die für das Tumorantigen MA-GE1 codierende mRNA modifiziert. Dadurch wurde die in Fig. 2C gezeigte Sequenz ermittelt, die im Vergleich zur Wildtyp-Sequenz (275 G, 244 G) einen um 24% höheren G/C-Gehalt aufweist (351 G, 291 C). Des weiteren wurde die Wildtyp-Sequenz durch alternative Codonverwendung hinsichtlich der Translationseffizienz aufgrund der Codierung von in der Zelle häufiger vorkommenden tRNAs verbessert (vgl. Fig. 2D). Durch die alternative Codonverwendung wurde der G/C-Gehalt ebenfalls um 24% erhöht.

### Anhang: Quelltext eines erfindungsgemäßen Computerprogramms

```
 Curevac_Genetic_Controls mit folgenden Modulen
 Name: Curevac_Genetic_Controls.vbp
 Code:
 Type=Control
 Reference=*\G{00020430-0000-0000-C000-000000000046}#2.0#0#.\.\WINNTSystem32\STB#OLE Automation
 Object={0D452EE1-E08F-101A-852E-02608CAD0BB4}#2.0#0; FM20.DLL
 Object={0D452EE1.E08F-101A-852E.02608C4D0BB4}# 2.0# 0; FM20.DLL
 Object={F9O43C88-F6F2-101A-A3C9-08002B2F49FB}# 1.2# 0; COMDLG32.OCX
 UserControl=Curevac_Amino.ctl
 Startup="(None)"
 HelpFile=""
 Title ="Curevac Genetic Controls"
 Command32=" "
 Name="Curevac_Genetic_Controls"
 HelpContextID="0"
 CompatibleMode="1"
 MajorVer=1
 MinorVer=0
 RevisionVer=0
 AutoIncrementVer=0
 ServerSupporFiles =0
 VersionComments="the RNA people"
 VersionCompanyName="CureVac GmbH"
 VersionFileDescription-"Controls for Handling of Nucleotids"
 VersionLegalCopyright="byChristian Klump"
 VersionLegalTrademarls="Guevac Genetic Controls(tm)"
 VersionProductName="Curevac Genetic Controls"
 CompilationType=0
 OptimizationType=0
 FavorPentiumPro(tm)=0
 CodeViewDebugInfo=0
 NoAliasing=0
 BoundsCheck=0
 OverflowCheck=0
 FlPointCheck=0
 FDIVCheck=0
 UnroundedFP=0
 StartMode=1
 Unattended=0
 Retained=0
 ThreadPerObject=0
 MaxNumberOfThreads=1
 ThreadingModel=1
 DebugStartupOption=1
 DebugStartupComponent=Curevac_Amino

 Name: Curevac_Amino.ctl
 Code:
 VERSION 5.00
 Object = "{0D452EE1-E08F-101A-852E-02608C4D0BB4}# 2.0# 0"; "FM20.DLL"
 Begin VB.UserControl Curevac_Amino
 CanGetFocus = 0 Talse
 ClientHeight = 690
 ClientLeft = 0
 ClientTop = 0
 ClientWidth = 1200
 ScaleHeight = 690
 ScaleWidth = 1200
 Begin VB.Line linLower
 X1 = 0
 X2 = 1080
 Y1 = 600
  Y2 = 600
 End
 Begin VB.Line linUpper
  X1 = 120
  X2 = 1080
  Y1 = 0
  Y2 = 0
 End
 Begin MSForms.Label lblAminoAcid
  DragMode = 1 'Automatic
  Height = 255
  Left = 120
  TabIndex = 1
  Top = 360
  Width = 975
  Size - "1720;450"
  SpecialEffect = 1
  FontName = "Lucida Sans Unicode"
  FontEffects = 1073741826
  FontHeight = 165
  FontCharSet = 0
  FontPitchAndFamily- 2
  ParagraphAlign = 3
 End
 Begin MSForms.Label lblTriplett
  DragMode = 1 'Automatic
  Height = 255
  Left = 120
 TabIndex = 0
  Top = 120
 Width = 975
  Size = "1720;450"
  SpecialEffect = 1
  FontHeight = 165
  FontChatSet = 0
  FontPitchAndFamily= 2
 ParagraphAlign = 3
  End
 End
 Attribute VB_Name = "Curevac_Amino"
 Attribute VB_GlobalNameSpace = False
 Attribute VB_Creatable = True
 Attribute VB_PredeclaredId = False
 Attribute VB_Exposed = True
 Option Explicit

 Private msAAShortcut As String
 Private msAAName As String
 Private msBestTriplett As String
 Private msSecondBest As String
 Private msThirdBest As String
 Private msTriplett As String
 Private msBackColor As Long
 Private mbShowOriginal As Boolean

 Public Enum enuAminoAcid
 amaGlycin
 amaAlanin
 amaValin
 amaLeucin
 amaIsoLeucin
 amaPhenylalanin
 amaTyrosin
 amaTryptophan
 amaAsparaginAcid
 amaAsparagin
 armGlutaminAcid
 amaGlutamin
 amaSerin
 amaThreonin
 amaCystein
 amaMethionin
 amaProlin
 amaHistidin
 amaLysin
 amaStop
 amaStart
 End Enum

 Private Sub Main()

 Call UserControl_Resize

 End Sub

 Public Sub Translation(ByVal sTriplett As String)

 msTriplett = sTriplett

 Select Case sTriplett
  Case "GGU", "GGC", "GGA", "GGG"
     msAAShortcut = "GLY"
  Case "GCU", "GCC", "GCA". "GCG"
     msAAShortcut = "ALA"
  Case "GUU", "GUC", "GUA", "GUG"
     msAAShortcut = "VAL"
  Case "UUC", "UUG", "CUU", "CUA", "CUG", "CUC"
     msAAShortcut = "LEU"
  Case "AUA", "AUU", "AUC"
     msAAShortcut = "ILE"
  Case "UUU", "UUC"
     nwAAShortcut - "PHE"
  Case "UAU", "UAC"
     msAAShortcut= "TYR"
  Case "UGG"
     msAAShortcut = "TRP"
  Case "GAU", "GAC"
     msAAShortcut = "ASP"
  Case "AAU", "AAC"
     msAAShortcut ="ASN"
  Case "GAA", "GAG"
     msAAShoitcut = "GLU"
  Case "CAA", "CAG"
    msAAshoncut m "GLN"
  Case "AGU", "AGC", "UCA", "UCU", "UCG", "UCC"
    msAAshortcut - "SER"
  Case "ACA", "AGU", "ACG", "AOC"
    msAAShortcut = "THR"
  Case "UGU", "UGC"
    msAAShortcut = "CYS"
  Case "AUG"
    msAAShortcut = "MET"
  Case "CCU", "COG", "COC"
     msAAShortcut = "PRO"
  Case "CAU", "CAC"
     msAAShortcut = "HIS"
   Case "AGA", "AGG", "CGA", "CGU", "CGG", "CGC"
     msAAShortcut = "ARG"
  Case "AAA", "AAG"
     msAAShortcut = "LYS"
  Case "UAA", "UAG", "UGA"
     msAAShortcut = "STP"
 End Select
 Call Synthesis(msAAShortcut)
 Call Show

 End Sub

 Public Sub Synthesis(ByVal sShortCut As String)

 msAAShortcut = sShortCut 'nur falls die Sub extern angesprochen wind

 Select Case msAAshorcut
  Case "GLY"
     msAAName ="Glycin"
     msBestTriplett = "GGC"
     msSecondBest = "GGG"
     msBackColor=8421631
  Case "ALA"
     msAAName ="Alanin"
     msBestTriplett = "GCG"
     msSecondBest ="GCC"
     msBackColor = 8454143
  Case "VAL"
     msAAName = "Valin"
     msBestTriplett = "GUC"
     msSecondBest = "GUG"
     msBackColor = 8454016
  Case "LEU"
     msAAName = "Leucin"
     msBestTriplett = "CUG"
     msSecondBest = "CUC"
     msBackColor = 16777088
  Case "ILE"
     msAAName ="Isoleucin"
     msBestTriplett = "AUC"
     msBackColor = 12615935
  Case "PHE"
     msAAName = "Phenylalanin"
     msBestTriplett = "UUC"
     msBackColor = 255
   Case "TYP"
     msAAName = "Tyrosin"
     msBestTriplett = "UAC"
     msBackColor = 4210816
   Case "TRP"
     msAAName = "Tryptophan"
     msBestTriplett = "UGG"
     msBackColor = 4227327
   Case "ASP"
     msAAName = "Asparaginsäure"
     msBestTriplett " "GAC"
     msBackColor = 8388863
   Case "ASN"
     msAAName = "Asparagin"
     msBestTriplett ="AAC"
     msBackColor = 4227200
   Case"GLU"
     msAAName = "Glutaminsäure"
     msBestTriplett = "GAG"
     msBackColor = 32768
   Case "GLN"
     msAAName = "Glutamin"
     msBestTriplett = "GGC"
     msBackColor = 8421440
   Case "SER"
     msAAName = "Serin"
     msBestTriplett = "AGC"
     msSecondBest = "UCG"
     msThirdBest = "UCC"
     msBackColor= 16512
   Case "THR"
     msAAName = "Threonin"
     msBestTriplett = "ACG"
     msBackColor =16711680
   Case "CYS"
     msAAName = "Cystein"
     msBestTriplett = "UGC"
     msBackColor = 6932960
   Case "MET"
     msAAName = "Methionin"
     msBestTriplett= "AUG"
     msBackColor = 10417643
   Case "PRO"
     msAAName = "Prolin"
     msBestTriplett= "CCG"
     msSecondBest = "CCC"
     msBackColor = 12898746
   Case "HIS"
     msAAName ="Histidin"
     msBestTriplett = "CAC"
     msBackColor = 12898746
   Case "ARG"
     msAAName = "Arginin"
     rmBestTriplett = "CGC"
     msSecondBest = "CGG"
     msBackColor = 6174925
   Case "LYS"
     msAAName = "Lysin"
     msBestTriplett = "AAG"
     msBackColor =14141641
  Case "STP"
      msAAName = "Stop"
     msBestTriplett - "UGA"
     msSecondBest = "UAG"
     msBackColor =11332093
 End Select

 End Sub

 Public Sub Show()

 lblAminoAcid.Caption = msAAShortcut
 If mbShowOriginal = True Then
   lblTriplett.Caption = msTriplett
 Else
   lblTriplett.Caption = msBestTriplett
 End If
 lblAminoAcid.BackColor = msBackColor
 lblTriplett.BackColor= msBackColor

 End Sub

 Public Function ChooseBestGC(sShortCut As String)

 Select Case sShortCut
   Case "GLY"
     msAAName = "Glycin"
     msBestTriplett ="GGC"
     msSecondBest = "GGG"
     msBackColor = 8421631
   Case "ALA"
     msAAName = "Alanin"
     msBestTriplett - "GCC"
     msSecondBest = "GCC"
     msBackColor = 8454143
   Case "VAL"
     msAAName = "Valin"
     msBestTriplett = "GUC"
     msSecondBest = "GUG"
     msBackColor = 8454016
   Case "LEU"
     msAAName = "Leucin"
     msBestTriplett = "CUG"
     msSecondBest = "CUC"
     msBackColor = 16777088
   Case "ILE"
     msAAName= "Isoleucin"
     msBestTriplett = "AUC"
     msBackColor = 12615935
   Case "PHE"
     msAAName = "Phenylalanin"
     msBestTriplett= "UUC"
     msBackColor = 255
   Case "TYR"
     msAAName = "TyroSin"
     msBestTriplett= "UAC"
     msBackColor = 4210816
   Case "TRP"
     msAAName = "tryptophan"
     msBestTriplett - "UGG"
     msBackColor= 4227327
   Case "ASP"
     msAAName = "Asparaginsäure"
     msBestTriplett = "GAC"
     msBackColor = 8388863
   Case "ASN"
     msAAName = "Asparagin"
     msBestTriplett = "AAC"
     msBackColor = 4227200
   Case "GLU"
     msAAName = "Glutaminsäure"
     msBestTriplett = "GAG"
     msBackColor = 16711808
   Case "GLN"
     msAAName = "Glutamin"
     msBestTriplett= "GGC'
     msBackColor = 12632256
   Case "SER"
     msAAName = "Serin"
     msBestTriplett= "AGC"
     msSecondBest = "UCG"
     msThirdBest = "UCC"
     msBackColor= 8421504
   Case "THR"
     msAAName = "Threonin"
     msBestTriplett= "ACG"
     msBackColor = &HD4O0FF
  Case "CYS"
     msAAName = "Cystein"
     msBestTriplett = "UGC"
     msBackColor = &HD5O0DFF
   Case "MET"
     msAAName = "Methionin"
     msBestTriplett = "AUG"
     msBackColor = &HD6O0FF
   Case "PRO"
     msAAName = "Prolin"
     msBestTriplett = "CCG"
     msSecondBest = "CCC"
     msBackColor = &HD7O0FF
   Case "HIS"
     msAAName = "Histidin"
     msBestTriplett = "CAC"
     msBackColor = &HD8C0FF
   Case "ARG"
     msAAName ="Argining"
     msBestTriplett = "CGC"
     msSecondBest = "CGG"
     nuBackColor = &HD9O0FF
     msAAName = "Lysin"
     msBestTriplett = "'AAG"
     msBackColor =&HE0C0DFF
   Case "STP"
     msAAName = "Stop"
     msBestTriplett = "UGA"
     msSecondBest = "UAG"
     msBackeColor = &HE1C0FF
  End Select

 End Function

 Public Property Let ShowOriginal(ByVal bNewValue As Boolean)
 mbShowOriginal = bNewValue
 End Property

 Public Property Get ShowOriginal() As Boolean
 ShowOriginal = mbShowOriginal
 End Property
 Public Property Get AAShortcut() As String
 AAShortcut = msAAShortcut
 End Property

 Public Property Get AAName() As String
 AAName = msAAName
 End Property

 Public Property Get BestTriplett() As String
 BestTriplett = msBestTriplett
 End Property

 Public Property Get SecondBest() As String
 SecondBest = msSecondBest
 End Property

 Public Property Get ThirdBest() As String
 ThirdBest =msThirdBest
 End Property

 Public Property Get BackColor() As String
 BackColor = msBackColor
 End Property

 Private Property Get Triplett() As String
 Triplett - msTriplett
 End Property

 Private Sub UserControl_Resize()

 Dim lOuterWidth As Long
 Dim lDistance As Long

 lDistance =30

 lOuterWidth = Scale Widh + lDistance

 With lblTriplett.
  .Top = 0
  .Left= lDistance / 2
  .Width = ScaleWidth - lDistance
  .Height = ScaleHeight / 2 + 30
 End With
 With lblAminoAcid
  .Top = ScaleHeight / 2 - 30    .Left = lDistance / 2
   .Width = ScaleWidth - lDistance
   Height = ScaleHeight / 2
 End Witth
 With linUpper
   .X1 = ScaleLeft
   .Y1 =0
   .X2 = Schale Width
   .Y2 =0
 End With
 With linLower
   .X1 = ScaleLeft
   .Y1= ScaleHeight - 20
   .X2 = Scale Width
   .Y2 = ScaleHeight - 20
 End With

 End Sub
```

Curevac_RNA_Optimizer mit folgenden Modulen

```
Name: Curevac_RNA_Optimizer.vbp
 Code:
 Type=Exe
 Reference=*\G{00020430-0000-0000-C000-
 000000000046}# 2.0# 0# ..\..\WINNT\System32\STDOLE2.TLB# OLE Automation
 Object-{F9043C88-F6F2-101A-A3C9-08002B2F49FB}# 1.2#0; COMDLG32.OCX
 Module= basMain; basMain.bas
 Form=mdiMain.frm
 Object=*\ACurevac_Genetic_Controls.vbp
 Object={38911DA0-E448-11D0-84A3-00DD01104159}# 1.1#0; COMCT332.OCX
 Object={3B7C8863-D78F-101B-B9B5-04021C009402}# 1.2#0; RICHTX32.OCX
 Form=frmOutPut.frm
 Form=frmStatistics.frm
 Module =basPublics; basPublics.bas
 Form=frmOptions.frm
 Form-frmInput.frm
 Form-frmDisplay.frm
 Form=frmAbout.frm
 Startup="mdiMain"
 HelpFile=""
 Title="RNA-Optimizer"
 Command32=""
 Name="RNA_Optimizer"
 HelpContextID="0"
 CompatibleMode="0"
 MajorVer=1
 MinorVer=0
 RevisionVer=0
 AutoIncrementVer=0
 ServerSupportFiles =0
 VersionComments="the RNA people"
 VersionCompanyName="CureVac GmbH"
 VersionFileDescription="Application for Optimization of RNA"
 VersionLegalCopyright="by Christian Klump"
 VersionLegalTrademarks="Curevac RNA-Optimizer(tm)"
 VersionProductName="Curevac RNA-Optimizer"
 CompilationType =0
 OptimizationType =0
 FavorPentiumPro(tm) =0
 CodeViewDebugInfo=0
 NoAliasing=0
 BoundsCheck=0
 OverflowCheck=0
 FIPointCheck=0
 FDIVCheck=0
 UnroundedFP=0
 StartMode=0
 Unattended=0
 Retained=0
 ThreadPerObject=0
 MaxNumberOfTreads=1
 DebugStartupOption=0

 Name: basMain.bas
 Code:
 Attribute VB_Name = "basMain"
 Option Explicit

 Private Type udtAminoAcid
 sShortcut As String
 sName As String
 sBestTriplett As String
 sSecondBest As String
 sThirdBest As String
 End Type

 Private mastrAminoAcids() As udtAminoAcid
 Public Sub RebuildChain(bShowOriginal As Boolean)
 Dim iLoop As Integer
 For iLoop =1 To glAminoCounter
  With frmDisplay.Curevac_Amino1(iLoop)
     .ShowOriginal = bShowOriginal
     .Show
  End With
 Next iLoop

 End Sub

 Public Sub ResetPublics()
 gsOriginalCode=""
 gsFormattedCode =" "
 glCodeLenght = ""
 End Sub

 Public Sub BuildAminoChain(ByVal sTempCode As String, bGraphical As Boolean)

 Dim lVerticalOffset As Long
 Dim.lHorizontalOffset As Long
 Dim lTop As Long

 Dim asTripletts() As String
 Dim sAminoChain As String
 Dim Loop As Long

 If bGraphical = True Then
  lTop = frmDisplay.optNucleotids(0).Height + 40
 End If
 ReDim mastrAminoAcids(glAminoCounter)
 asTripletts = Split(sTempCode)
 For lLoop =0 To glAminoCounter- 1
 If bGraphical = True Then
     Load frmDisplay.Curevac_Amino1(lLoop +1)
     With frmDisplay.Curevac_Amino1(1Loop + 1)

       Call. Translation(asTripletts(ILoop))

       .Left = lLoop* Width - lHorizontalOffset
       .Top = lVerticalOffset + lTop
       If .Left + 2*.Width > frmDisplay.ScaleWidth Then
         IVerticalOffset = lVerticalOffset + .Height + 150
         lHorizontalOffset - ILoop* With + .Width
       End If

       sAminoChain = sAminoChain + .aaShortcut + ","
      .Visible - True
     End With
   Else
     mastrAminoAcids(ILoop) = Translation(asTripletts(lLoop))
      sTest = sTest + mastrAminoAcids(iLoop).sName + ";"
   End If
 Next
 If bGraphical = True Then
   frmDisplayShow
 Else
   Call frmOutPut.Show
 End If
 Debug.Print sAminoChain
 End Sub

 Private Function Translation(ByVal sTriplett As String) As udtAminoAcid

 Dim stiTemp As udtAminoAcid

 If mdiMain.optKindOfOptimize(0) = True Then
   'Optimization for Most GC
   Select Case sTriplett
     Case "GGU", "GGC", "GGA", "GGG"
       stiTemp.sShortcut = "GLY"
       stiTemp.sName = "Glycin"
       stiTemp.sBestTriplett = "GGC
       stiTemp.sSecondBest = "GGG"
     Case "GCU", "GCC", "GCA", "GCG"
     stiTemp.sShortcut= "ALA"
       stiTemp.sName = "Alanin"
       stiTemp.sBestTriplett = "GCG"
       stiTemp.sSecondBest = "GCC"
     Case "GUU", "GUC", "GUA" , "GUG"
       stiTemp.sShortcut = "VAL"
       sitTemp.sName ="Valin"
       stiTemp.sBestTriplett = "GUC"
       stiTemp.sSecondBest = "GUG"
     Case "UUA", "UUG"; "CUU", "CUA", "CUG", "CUC"
       stiTemp.sShortcut = "LEU"
       stiTemp.sName = "Leucin"
       stiTemp.sBestTriplett = "CUG"
       stiTemp.sSecondBest = "CUC"
     Case "AUA", "AUU", "AUC"
       stiTemp.sShortcut= "ILE"
       stiTemp.sName = "Isoleucin"
       stiTemp.sBestTriplett = "AUC"
     Case "UUU", "UUC'
       stiTemp.sShortcut = "PHE"
       stiTemp.sName= "Phenylalanin"
       stiTemp.sBestTriplett = "UUC"
     Case "UAU", "UAC"
       stiTemp.sShortcut = "TYR"
       sti"Temp.sName = "Tyrosin"
       stiTemp.sBestTriplett = "UAC"
     Case "UGG"
       stiTemp.Shortcut = "TRP"
       stiTemp.sName = "Tryptophan"
       stiTemp.sBestTriplett = "UGG"
     Case "GAU", "GAC"
       stiTemp.sShortcut = "ASP"
       stiTemp.sName = "Asparaginsäure"
       sriTemp.sBestTriplett = "GAC"
     Case "AAU", "AAC"
       stiTemp.sShortcut = "ASN"
       stiTemp.sName = "Asparagin"
       stiTemp.sBestTriplett = "AAC"
     Case "GAA", "GAG"
       stiTemp.sShortcut = "GLU"
       stiTemp.sName = "Glutaminsäure"
       stiTemp.sBestTriplett = "GAG"
     Case "CAA", "CAG"
      stiTemp.sShortcut = "GLN"
       stiTemp.sName = "Glutamin"
       stiTemp.sBestTriplett = "CAG"
     Case "AGU", "AGC", "UCA", "UCU", "UCG", "UCC"
       stiTemp.sShortcut = "SER"
       stiTemp.sName = "Serin"
       stiTemp.sBestTriplett = "AGC"
       stiTemp.sSecondBest = "UCG"
       stiTemp.sThirdBest = "UCC"
     Case "ACA", "ACU", "ACG", "ACC"
       stiTemp.sShortcut = "THR"
       stiTemp.sName = "Threonin"
       stiTemp.sBestTriplett = "ACG"
     Case "UGU", "UGC"
       stiTemp.sShortcut = "CYS"
       stiTemp.sName = "Cystein"
       stiTemp.sBestTriplett = "UGC"
     Case "AUG"
       stiTemp.sShorcut = "MET"
       stiTemp.sName = "Methionin"
       stiTemp.sBestTriplett = "AUG"
     Case "CCA", "OCU", "CCG", "CCC"
       stiTemp.sShortcut = "PRO"
       stiTemp.sName = "Prolin"
       stiTemp.sBestTriplett = "CCG"
       stiTemp.sSecondBest = "CCC"
     Case "CAU", "CAC"
       stiTemp.Shortcut = "HIS"
       stiTemp.sName = "Histidin"
       stiTemp.sBestTriplett = "CAC"
     Case "AGA", "AGG", "CGA", "CGU", "CGG", "CGC"
       stiTemp.sShortcut = "ARG"
       stiTemp.sName = "Arginin"
       stiTemp.sBestTriplett = "CGC"
       stiTemp.sSecondBest = "CGG"
     Case "AAA", "AAG"
       stiTemp.sShorccut = "LYS"
       stiTemp.sName = "Lysin"
       stiTemp.sBestTriplett = "AAG"
     Case "UAA", "UAG", "UGA"
       stiTemp.sShortcut = "STP"
       stiTemp.sName = "Stop"
       stiTemp.sBestTriplett = "UGA"
       stiTemp.sSecondBest = "UAG"
   End Select
  Translation = stiTemp
 Else 'Optimization for best frequency
   Select Case sTriplett
     Case "GGU", "GGC", "GGA", "GGG"
       stiTemp.sShortcut = "GLY"
       stiTemp.sName = "Glycin"
       stiTemp.sBestTriplett = "GGC"
       stiTemp.sSecondBest = "GGU"
     Case "GCU", "GCC", "GCA", "GCG"
       stiTemps.Shortcut = "ALA"
       stiTemp.sName = "Alanin"
       stiTemp.sBestTriplett = "GCC"
       stiTemp.sSecondBest = "GCU"
     Case "GUU", "GUC", "GUA", "GUG"
       stiTemp.sShortcut = "VAL"
       stiTemp.sName = "Valin"
       stiTemp.sBestTriplett = "GUG"
       stiTemp.sSecondBest = "GUC"
     Case "UUA", "UUG", "CUU", "CUA", "CUG", "CUC"
       stiTemp.sShortcut = "LEU"
       stiTemp.sName = "Leucin"
       stiTemp.sBestTriplett = "CUG"
       stiTemp.sSecondBest = "CUC"
     Case "AUA", "AUU", "AUC"
       stiTemp.sShortcut = "ILE"
       stiTemp.sName = "Isoleucin"
       stiTemp.sBestTriplett = "AUC"
     Case "UUU", "UUC"
       stiTemp.sShortcut = "PHE"
       sriTemp.sName = "Phenylalanin"
       stiTemp.sBestTriplett = "UUC"
     Case "UAU", "UAC"
       stiTemp.sShortcut = "TYR"
       stiTemp.sName = "Tyrosin"
       stiTemp.sBestTriplett = "UAC"
     Case "UGG"
       stiTemp.sShortcut = "TRP"
       stiTemp.sName = "Tryptophan"
       stiTemp.sBestTriplett = "UGG"
     Case "GAU", "GAC"
       stiTemp.sShortcut = "ASP"
       stiTemp.sName = "Asparaginsäure"
       stiTemp.sBestTriplett = "GAC"
     Case "AAU", "AAC"
       stiTemp.sShortcut = "ASN"
       stiTemp.sName = "Asparagin"
       stiTemp.sBestTriplett = "AAC"
     Case "GAA", "GAG"
       stiTemp.sShortcut = "GLU"
       stiTemp.sName = "Glutaminsäure"
       stiTemp.sBestTriplett = "GAG"
     Case "CAA", "CAG"
       stiTemp.sShortcut = "GLN"
       stiTemp.sName = "Glutamin"
       stiTemp.sBestTriplett = "CAG"
     Case "AGU", "AGC", "UCA", "UCU", "UCG", "UCC"
       stiTemp.sShortcut = "SER"
       stiTemp.sName = "Serin"
       stiTemp.sBestTriplett = "AGC"
       stiTemp.sSecondBest = "UCC"
       stiTemp.sThirdBest = "UCU"
     Case "ACA", "ACU", "ACG", "ACC"
       stiTemp.sShortcut = "THR"
       stiTemp.sName = "Threonin"
       stiTemp.sBestTriplett = "ACC"
     Case "UGU", "UGC"
       stiTemp.sShortcut = "CYS"
       stiTemp.sName = "Cystein"
       stiTemp.sBestTriplett = "UGC"
     Case "AUG"
       stiTemp.sShortcut = "MET"
       stiTemp.sName = "Methionin"
       stiTemp.sBestTriplett = "AUG"
     Case "CCA", "CCU", "CCG", "CCC"
       stiTemp.sShortcut = "PRO"
       stiTemp.sName = "Prolin"
       stiTemp.sBestTriplett = "CCC"
       stiTemp.sSecondBest = "CCU"
     Case "CAU", "CAC"
       stiTemp.sShortcut = "HIS
       stiTemp.sName = "Histidin"
       stiTemp.sBestTriplett = "CAC'
    Case "AGA", "AGG", "CGA", "CGU", "CGG", "CGC"
       stiTemp.sShorcut = "ARG"
       stiTemp.sName = "Arginin"
       stiTemp.sBestTriplett = "CGC"
       stiTemp.sSecondBest = "AGG"
    Case "AAA", "AAG"
       stiTemp.sShortcut = "LYS"
       stiTemp.sName = "Lysin"
       stiTemp.sBestTriplett - "AAG"
    Case "UAA", "UAG", "UGA"
       stiTemp.sShortcut = "STP"
       stiTemp.sName = "Stop"
       stiTemp.sBestTriplett = "UGA"
       stiTemp.sSecondBest = "UAG"
  End Select
  Translation = stiTemp
 End If

 End Function

 Public Function ReverseTranscription() As String
 Dim sTempCode As String
 Dim iLoop As Integer
 For iLoop = 0 To glAminoCounter - 1
  sTempCode = sTempCode + mastrAminoAcids(iLoop).sBestTriplett
 Next
 ReverseTranscription = sTempCode
 End Function

 Public Sub CountBases(ByVal sTempCode As String)

 Dim lLoop As Long
 Dim sFormattedCode As String
 Dim sActualBase As String
 Dim lCodeLength As Long

 lCodeLength = Len(sTempCode)
 For lLoop =1 To CLng(lCodeLength)
  sActualBase = Mid(sTempCode, lLoop, 1)
  Select Case sActualBase
    Case "A", "a"
       glOptAdenin = glOptAdenin + 1
    Case "U", "u", "T", "t"
      glOptThymin = glOptThymin + 1
    Case "G", "g"
      glOptGuanin = glOptGuanin + 1
    Case "C", "c"
      glOptCytosin = glOptCytosin + 1
  End Select
 Next lLoop
 End Sub

 Name: basPublics.bas
 Code:
 Attribute VB_Name = "basPublics"
 Option Explicit

 Public gsOriginalCode As String
 Public gsFormattedCode As String
 Public glCodeLength As Long

 Public glAaminoCounter As Long
 Public glAdenin As Long
 Public glThymin As Long
 Public glGuanin As Long
 Public glCytosin As Long
 Public glOptAdenin As Long
 Public glOptThymin As Long
 Public glOptGuanin As Long
 Public glOptCytosin As Long

 Public gbSequenceChanged As Boolean

 Name: mdiMain.frm
 Code:
 VERSION 5.00
 Object = "{38911DA0-E448-11D0-84A3-00DD01104159}# 1.1#0"; "COMCT332.OCX"
 Begin VB.MDIForm mdiMain
 BackColor = &H00FFFFFF&
 Caption = "Curevac_RNA_Analyzer"
 ClientHeight = 8745
 ClientLeft = 165
 ClientTop = 735
 ClientWidth = 12255
 Icon = "mdiMain.frx":0000
 LinkTopic = "MDIForm1"
 Picture = "mdiMain.frx":058A
 StartUpPosition = 3 'Windows Default
 WindowState = 2 'Maximized
 Begin ComCrl3.CoolBar cbarMain
  Align = 1 'Align Top
  Height = 885
  Left = 0
  TabIndex = 0
  Top = 0
  Width = 12255
  _ExtentX = 21616
  _ExtentY = 1561
  BandCount = 1
  BandBorders = 0 'False
  _CBWidth = 12255
  _CBHeight = 885
  _Version = "6.7.8988"
  MinHeight1 = 825
  Width1 = 4995
  FixedBackgound1= 0 'False
  NewRow1 = 0 'False
  Begin VB.OptionButton optKindOfOptimize
   Caption = "Best Frequency"
   Height = 255
   Index = 1
   Left = 4920
   TabIndex = 7
   Top = 480
   Value = -1 True
   Width = 1575
  End
  Begin VB.OptionButton optKindOfOptimize
   Caption = "Best GC"
   Height = 255
   Index = 0
   Left = 4920
   TabIndex = 6
   Top = 120
   Width = 1575
  End
  Begin VB.CommandButton cmdShowInput
   Caption = "Input"
   Height = 765
   Left = 0
   Picture = "mdiMain.frx":0C2A
   Style = 1 'Graphical
   TabIndex = 5
   Top = 0
   Width = 840
  End
  Begin VB.CommandButton cmdShowOptions
   Caption = "Option"
   Enabled = 0 'False
   Height = 760
   Left = 3480
   Picture = "mdiMain.frx":0F34
   Style = 1 'Graphical
   TabIndex = 4
   Top = 0
   Width = 735
  End
  Begin VB.CommandButton cmdShowStatistics
   Caption = "Statistics"
   Enabled = 0 'False
   Height = 760
   Left = 2640
   Picture = "mdiMain.fx":131E
   Style = 1 'Graphical
   TabIndex = 3
   Top = 0
   Width = 735
  End
  Begin VB.CommandButton cmdShowDisplay
   Caption = "Display"
   Enabled = 0 'False
   Height = 760
   Left = 1800
   Picture = "mdiMain.frx":1628
   Style = 1 'Graphical
   TabIndex = 2
   Top = 0
   Width = 735
 End
 Begin VB.CommandButton cmdShowOutput
   Caption = "Output"
   Enabled = 0 'False
   Height = 760
   Left = 960
   Picture = "mdiMain.frx":20E2
   Style = 1 'Graphical
   TabIndex = 1
   Top = 0
   Width = 735
 End
 End
 Begin VB.Menu mnuMain
 Caption = "&Input..."
  Index = 0
 End
 Begin VB.Menu mnuMain
  Caption = "&Results"
  Index = 1
  Begin VB.Menu mnuResults
   Caption = "&Output..."
   Enabled = 0 'False
   Index = 0
  End
  Begin VB.Menu mnuResults
   Caption = "&Display..."
   Enabled = 0 'False
   Index = 1
  End
  Begin VB.Menu mnuResults
   Caption = "&Statistics..."
   Enabled = 0 'False
   Index = 2
  End
 End
 Begin VB.Menu mnuMain
  Caption = "E&xtras"
  Index = 4
  Begin VB.Menu mnuExtras
   Caption = "&Language"
   Index = 0
   Begin VB.Menu mnuLanguage
     Caption = "English"
     Checked = -1 True
     Index = 0
   End
   Begin VB.Menu mnuLanguage
     Caption = "&German"
     Enabled = 0 'False
     Index = 1
   End
   Begin VB.Menu mnuLanguage
     Caption = "&French"
     Enabled = 0 'False
     Index = 2
   End
  End
  Begin VB.Menu mnuExtras
   Caption = "&Options..."
   Enabled = 0 'False
   Index = 1
  End
  Begin VB.Menu mnuExtras
   Caption = "-"
   Index = 2
  End
  Begin VB.Menu mnuExtras
   Caption = "&About"
   Index = 3
 End
 End
 Begin VB.Menu mnuMain
 Caption = "&Windows"
 Index = 5
 WindowList = -1 True
 Begin VB.Menu mnuWindows
   Caption = "Tile &Horizontally"
   Index = 0
 End
 Begin VB.Menu nmuWindows
   Caption = "Tile &Vertically"
   Index = 1
 End
 Begin VB.Menu mnuWindows
   Caption = "&Cascade"
   Index = 2
 End
 End
 Begin VB.Menu mnuMain
 Caption = "&Exit"
 Index = 6
 End
 End
 Attribute VB_Name = "mdiMain"
 Attribute VB_GlobalNameSpace = False
 Attribute VB_Creatable = False
 Attribute VB_PredeclaredId = True
 Attribute VB_Exposed = False
 Option Explicit

 Private Sub cmdShowDisplay_Click()
 Call frmDisplay.Show
 End Sub

 Private Sub cmdShowInput_Click()
 Call frmInput.Show
 End Sub

 Private Sub cmdShowOptions_Click()
 Call frmOptions.Show
 End Sub

 Private Sub cmdShowOutput_Click()
 Call frmOutPut.Show
 End Sub

 Private Sub cmdShowStatistics_Click()
 Call frmStatistics.Show
 End Sub

 Private Sub MDIForm_Load()

 Call InitControls
 Call frmInput.Show
 End Sub

 Private Sub InitControls()
 Dim lNextLeft As Long
 Const lSpace As Long = 60
 Const lButtonWidth As = 850
 Const lButtonHeight As Long = 770

 lNextLeft = lSpace + lButtonWidth

 With cmdShowInput
   .Top = lSpace
  .Left = lSpace
  .Width = lButtonWidth
   .Height = lButtonHeight
 End With

 With cmdShowOutput
   .Top = lSpace
  .Left = lNextLeft + lSpace
  .Width = lButtonWidth
  .Height = lButtonHeight
 End With

 With cmdShowDisplay
  .Top = lSpace
  .Left = lNextLeft * 2 + lSpace
  .Width = lButtonWidth
  .Height = lButtonHeight
 End With

 With cmdShowStatistics
  .Top = lSpace
  .Left = lNextLeft * 3 + lSpace
   .Width = IButtonWidth
   .Height = IButtonHight
 End With

 With cmdShowOptions
 .Top = ISpace
   .Left = INextLeft * 4 + lSpace
   .Width = IButtonWidth
   .Height = IButtonHight
 End With

 cbarMain.Height = IButtonHeight + 2 * Ispace

 End Sub

 Private Sub mnuMain_Click(Index As Integer)

 Select Case Index
   Case 0
     Call frmInput.Show
   Case 6
     Unload Me
 End Select

 End Sub

 Private Sub mnuResults_Click(Index As Integer)

 Select Case Index
   Case 0 'Output
     Call frmOutPut.Show
   Case 1
     Call frmDisplay.Show
   Case 2 'Statistische Auswertung
     Call frmStatistics.Show
 End Select

 End Sub

 Private Sub mnuExtras_Click(Index As Integer)

 Select Case Index
   Case 0 'Output
  Case 1
     Call frmDisplay.Show
  Case 3
     Call frmAbout.Show(vbModal)
 End Select

 End Sub

 Private Sub mnuWindows_Click(Index As Integer)

 Select Case Index
   Case 0 'Horizontal
     Me.Arrange (vbTileHorizontal)
   Case 1 'Vertikal
     Me.Arrange (vbTileVertical)
   Case 2 'Kaskadieren
     Me.Arrange (vbCascade)
 End Select

 End Sub

 Name: frmInput.frm
 Code:
 VERSION 5.00
 Object = "{F9043C88-F6F2-101A-A3C9-08002B2F49FB}# 1.2# 0"; "COMDLG32.OCX"
 Object = "{3B7C8863-D78F-101B-B9B5-04021C009402}# 1.2#0"; "RICHTX32.OCX"
 Begin VB.Form frmInput
 Caption = "Input"
 ClientHeight = 5730
 ClientLeft = 60
 ClientTop = 345
 ClientWidth = 13200
 Icon = "frmInput.frx":0000
 LinkTopic = "Form1"
 MDIChild = -1 True
 ScaleHeight = 5730
 ScaleWidth = 13200
 WindowState = 2 'Maximized
 Begin RichTextLib.RichTextBox txtFormatted
 Height = 1815
 Left = 120
 TabIndex = 3
 Top = 360
 With = 12735
 _ExtentX = 22463
 _ExtentY = 3201
 _Version = 393217
 Bordetstyle = 0
 Enabled = -1 True
 ScrollBars = 2
 DisableNoScroll = -1 True
 TextRTF = $"frmInput.frx":030A
 BeginPropertyFont {0BE35203-8F91-11CE-9DE3-00AA004BB851}
   Name = "Fixedsys"
   Size = 9
   Charset = 0
   Weight = 400
   Underline = 0 'FaLse
   Italic = 0 'False
   Strikethrough = 0 'False
 EndProperty
 End
 Begin VB.OptionButton optSequence
 Caption = "Formatted"
 Height = 255
 Index = 1
 Left = 2760
 Style = 1 'Graphical
 TabIndex = 2
 Top = 0
 Value = -1 True
 Width = 855
 End
 Begin VB.OptionButton optSequence
 Caption = "Original"
 Height = 255
 Index = 0
 Left = 1920
 Style = 1 'Graphical
 TabIndex = 1
 Top = 0
 Width = 855
 End
 Begin VB.CommandButton cmdLoad
 Caption = "Load Sequence"
 Height = 285
 Left = 0
 TabIndex = 0
 Top = 0
 Width = 1695
 End
 Begin MSComDlg.CommonDialog CommonDialog1
 Left = 3840
 Top = -120
 _ExtentX = 847
 _ExtentY = 847
 _Version = 393216
 End
 End
 Attribute VB_Name = "frmInput"
 Attribute VB_GlobalNameSpace = False
 Attribute VB_Creatable = False
 Attribute VB_PredeclaredId = True
 Attribute VB_Exposed = False
 Option Explicit

 Private Sub Form_Activate()
 Me.ZOrder
 Call InitControls
 End Sub

 Public Sub LoadSequence()

 Dim sFile As String
 Dim sTempCode As String

 On Error Go To Errorhandler
 With CommonDialog1
   .FileName = App.Path & "\SampleRNA.txt"
   .CancelError = True
   Call.ShowOpen
 End With
 If Len(CommonDialog1.FileName) >0. Then
  gbSequenceChanged = True
  gsFormattedCbde=""
  gsOriginalCode = ""
  sFile = CommonDialog1.FileName
   Open sFile For Input As # 1
  Input # 1, gsOriginalCode
   Close # 1
  Call FormatText
  Call FillSequence

  mdiMain.cmdShowDisplay.Enabled = True
  mdiMain.cmdShowOutput.Enabled = True
  mdiMain.cmdShowStacistics.Enabled = True
 End If

 Exit Sub

 Errorhanler:

 Dim IAnswer As Long
 If ErrNumber <>cdlCancel Then
   lAnswer = MsgBox("Datei konnte nicht geladen werden", vbRetryCancel, "Dateiprob-
 lem")
   If IAnswer = vbRetry Then
     Resume
   End If
 End If

 End Sub

 Private Sub FormatText()

 Dim iLoop As Integer
 Dim sActualBase As String

 glCodeLength = Len(gsOriginalCode)
 For iLoop = 0 To glCodeLength - 1
   sActualBase = Mid(gsOriginalCode, iLoop +1,1)
   If iLoop Mod 3 = 0 Then
     gsFormattedCode = gsFormattedCode + " "
     glAminoCounter = glAminoCounter + 1
   End If
   Select Case sActualBase
     Case "A", "a"
       gsformattedCode = gsFormattedCode + "A"
       glAdenin = glAdenin + 1
     Case "U", "u", "T", "t"
       gsformattedCode = gsformattedCode + "U"
       glThymin = glThymin + 1
     Case "G", "g"
       gsFormattedCode = gsFormattedCode + "G"
       glGuanin = glGuanin + 1
     Case "C", "c"
       gsFormattedCode = gsFormattedCode + "C"
       glCytosin = glCytosin + 1
   End Select
 Next iLoop
 gsFormattedCode = Trim(gsFormattedCode)

 End Sub

 Private Sub FillSequence()
 If optSequence(0).Value = True Then
   txtFormatted.Text = gsOriginalCode
 Else
  txtFormatted.Text = gsFormattedCode
 End If
 End Sub

 Private Sub cmdShowOutput_Click()
 Call frmOutPut.Show
 End Sub

 Private Sub cmdLoad_Click()
 Call LoadSequence
 End Sub

 Private Sub InkControls()

 ConstISpace As Long = 40
 Const IButtonHeight As Long = 285

 With cmdLoad
  .Top = ISpace
  .Height = IButtonHight
  .Left = Ispace
 End With

 With optSequence(0)
  .Top = ISpace
  .Height = IButtonHeight
  .Left = cmdLoad.Left + cmdLoad.Width + ISpace + 200
 End With

 With optSequence(1)
  .Top = ISpace
  .Height = IButtonHight
  .Left = optSequence(0).Left + optSequence(0).Width
 End With

 With txtFormatted
  .Top = cmdLoad.Height + 2 * ISpace
  .Left = lSpace
  .Width = Me.ScaleWidth
  .Height = Me.ScaleHeight - txtFomatted.Top
 End With
 End Sub

 Private Sub optSequence_Click(Index As Integer)
 Call FillSequence
 End Sub

 Name: frmOutPut.frm
 Code:
 VERSION 5.00
 Object = "{3B7C8863-D78F-101B-B9B5-04021C009402}# 1.2# 0"; "RICHTX32.OCX"
 Begin VB.Form frmOutPut
 Caption = "OutPut"
 ClientHeight = 9120
 ClientLeft = 60
 ClientTop = 345
 ClientWidth = 10215
 Icon = "frmOutPut.frx":0000
 LinkTopic = "Form1"
 MDIChild = -1 True
 ScaleHeight = 9120
 ScaleWidth = 10215
 Begin RichTextLib.RichTextBox txtOptimized
  Height = 2655
  Left = 0
  TabIndex = 0
  Top = 480
  Width = 9855
  _ExtemX = 17383
 _ExtentY = 4683
 _Version = 393217
 Enabled = -1 True
  ScrollBars = 2
 DisableNoScroll = -1 True
  TextRTF = $"frmOutPut.frx":0442
 BeginPropertyFont {0BE35203-8F91-11CE-9DE3-00AA004BB851}
   Name = "Fixedsys"
   Size = 9
   Charset = 0
   Weight = 400
   Underline = 0 'False
   Italic = 0 'False
   Strikethrough = 0 'False.
 EndProperty
 End
 End
 Attribute VB_Name = "frmOutPut"
 Attribute VB_GlobalNameSpace = False
 Attribute VB_Creatable = False
 Attribute VB_Predeclaredld = True
 Attribute VB_Exposed = False
 Option Explicit

 Private Sub Form_Activate()

 Me.ZOrder
 Call InitControls
 If gbSequenceChanged = True Then
   Call BuildAminoChain(gsFormattedCode, Falte)
 End If
 gbSequenceChanged = False
 txtOptimized.Text = LCase(ReverseTranscription)

 End Sub

 Private Sub InitControls()

 Const ISpace As Long = 40

 With txtOptimized
   .Top = ISpace
   .Left = ISpace
   .Width = Me.ScaleWidth + ISpace
   .Height = Me.ScaleHeight + Ispace
 End With
 End Sub

 Name: frmDisplay.frm
 Code:
 VERSION 5.00
 Object = "*\ACurevac_Genetic_Controls.vbp"
 Begin VB.Form frmDisplay
 Caption = "Display"
 ClientHeight = 8205
 ClientLeft = 60
 ClientTop = 345
 ClientWidth = 8745
 Icon = "frmDisplay.frx":0000
 LinkTopic = "Form1"
 MDIChild = -1 True
 ScaleHeight = 8205
 ScaleWidth = 8745
 ShowInTaskbar = 0 'False
 Begin VB.OptionButton optNucleotids
  Caption = "Original"
  Height = 255
  Index = 0
  Left = 0
  Style = 1 'Graphical
  TabIndex = 1
  Top = 0
  Width = 855
 End
 Begin VB.OptionButton optNucleotids
  Caption = "Optimized"
  Height = 255
  Index = 1
  Left = 840
  Style = 1 'Graphical
  TabIndex = 0
  Top = 0
  Value = -1 True
  Width = 855
 End
 Begin Curevac_Genetic_Controls.Curevac_Amino Curevac_Amino1
  Height = 495
  Index = 0
  Left = 240
  Top = 360
  Visible = 0 'False
  Width 735
  _ExtentX = 1296
  _ExtentY = 873
 End
 End
 Attribute VB_Name = "frmDisplay"
 Attribute VB_GlobalNameSpace = False
 Attribute VB_Creatable = False
 Attribute VB_PredeclaredId = True
 Attribute VB_Exposed = False
 Option Explicit

 Private Sub Form_Activate()

 Me.ZOrder
 If gbSequenceChanged = True Then
   Call BuildAminoChain(gsFormattedCode, True)
 End If
 gbSequenceChanged = False

 End Sub

 Private Sub optNucleotids_Click(Index As Integer)

 Dirn bShowOriginal As Boolean

 If Index = 0 Then 'Original Clicked
   bShowOriginal = True
 Else 'Optimized Clicked
   bShowOriginal = False
 End If
 Call RebuildChain(bShowOriginal)

 End Sub

 Name: frmStatistics.frm
 Code:
 VERSION 5.00
 Begin VB.Form frmStatistics
 Caption = "Statistics"
 ClientHeight = 6450
 ClientLeft = 60
 ClientTop = 345
 ClientWidth = 8595
 Icon = "frmStatistics.frx":0000
 LinkTopic = "formy1"
 MDIChild = -1 True
 ScaleHeight = 6450
 ScaleWidth = 8595
 Begin VB.Frame Frame1
 Caption = original"
 Height = 2655
 Left = 120
 TabIndex = 9
 Top = 720
 Width = 2175
 Begia VB.Label lblAdenin
   BorderStyle = 1 'Fixed Single
   Height = 495
   Left = 840
   TabIndex = 17
   Top = 240
   Width = 1215
 End
  Begin VB.Label lblCytosin
   BorderStyle = 1 'Fixed Single
   Height = 495
   Left = 840
   TabIndex = 16
   Top = 2040
   Width = 1215
  End
  Begin VB.Label lblGuanin
   BorderStyle = 1 'Fixed Single
   Height = 495
   Left = 840
   TabIndex = 15
   Top = 1440
   Width = 1215
  End
  Begin VB.Label lblThymin
   BorderStyle = 1 'Fixed Single
   Height = 495
   Left = 840
   TabIndex = 14
   Top = 840
   Width = 1215
  End
  Begin VB.Label Label14
   Caption = "Cytosin"
   Height = 255
   Left = 120
   TabIndex = 13
   Top = 2160
   Width = 615
  End
  Begin VB.label Label2
   Caption = "Thymin"
   Height = 255
   Left = 120
   TabIndex = 12
   Top = 960
   Width = 615
  End
  Begin VB.Label Label3
   Caption = "Guanin"
   Height = 255
   Left = 120
   TabIndex = 11
   Top = 1560
   Width = 615
  End
  Begin VB.Label Label1
   Caption = "Adenin"
   Height = 255
   Left = 120
   TabIndex = 10
   Top = 360
   Width = 615  End
 End
 Begin VB.Frame Frame2
 Caption = "optimized"
 Height = 2655
 Left = 2520
 TabIndex = 0
 Top = 720
 Width = 2175
  Begin VB.Label Label4
   Caption = "adenin"
   Height = 255
   Left = 120
   TabIndex = 8
   Top = 360
   Width = 615
  End
  Begin VB.Label Label6
   Caption = "Cytosin"
   Height = 255
   Left = 120
   TabIndex = 7
   Top = 2160
   Width = 615
  End
  Begin VB.Label Label8
   Caption = "Guanin"
   Height = 255
   Left = 120
   TabIndex = 6
   Top = 1560
   Width = 615
  End
  Begin VB.Label Label12
   Caption = "Thymin"
   Height = 255
   Left = 120
   TabIndex = 5
   Top = 960
   Width = 615
  End
  Begin VB.Label lblOptAdenin
   BorderStyle = 1 'Fixed Single
   Height = 495
   Left = 840
   TabIndex = 4
   Top = 240
   Width = 1215
  End
  Begin VB.Label lblOptThymin
   BorderStyle = 1 'Fixed Single
   Height = 495
   Left = 840
   TabIndex = 3
   Top = 840
   Width = 1215
  End
  Begin VB.Label lblOptGuanin
   BodetSty4e = 1 'Fixed Single
   Height = 495
   Left = 840
   TabIndex = 2
   Top = 1440
   Width = 1215
  End
  Begin VB.Label lblOptCytosin
   BorderStyle = 1 'Fixed Single
   Height = 495
   Left = 840
   TabIndex = 1
   Top = 2040
   Width = 1215
  End
 End
 Begin VB.Label IblBases
  BordetStyle = 1 'Fixed Single
  Height = 495
  Left = 1320
  TabIndex = 19
  Top = 120
  Width = 1215
 End
 Begin VB.Label Label5
  Caption = "Sum of bases"
  Height = 255
  Left = 120
  Tablndex = 18
  Top = 240
  Width = 1095
 End
 End
 Attribute VB_Name = "frmStatistics"
 Attribute VB_GlobalNameSpace = False
 Attribute VB_CReatable = False
 Attribute VB_PredeclaredId = True
 Attribute VB_Exposed = False
 Option Explicit
 Private Sub Form_Activate()
 Me.ZOrder

 lblBases.Caption = CStr(glAminoCounter * 3)
 lblAdenin.Caption = CStr(glAdenin)
 lblThymin.Caption = CStr(glThymin)
 IblGuanin.Caption = CStr(glGuanin)
 lblCytosin.Caption = CStr(glCyrosin)

 Call CountBases(frmOutPut.txtOptimized.Text)
 lblCptAdeninoCaption = CStr(glOptAdenin)
 lblOptThymin.Caption = CStr(glOptThymin)
 lblOptGuanin.Caption = CStr(glOptGuanin)
 lblOptCytosin.Caption = CStr(glOptCytosin)

 End Sub

 Name: frmOptions.frm
 Code:
 VERSION 5.00
 Begin VB.Form frmOptions
 Caption = "Options"
 ClientHeight = 3915
 ClientLeft = 60
 ClientTop = 345
 ClientWidth = 5760
 Icon = "frmOptions.frx":0000
 LinkTopic = "Form1"
 MDIChild = -1 True
 ScaleHeight = 3915
 ScaleWidth = 5760
 End
 Attribute VB_Name = "frmOptions"
 Attribute VB_GlobalNameSpace = False
 Attribute VB_Creatable = False
 Attribute VB_PredeclaredId = True
 Attribute VB_Exposed = False
 Option Explicit

 Name: frmAbout.frm
 Code:
 VERSION 5.00
 Begin VB.Form frmAbout
 BorderStyle = 3 'Fixed Dialog
 Caption = "About Curevac RNA-Analyzer"
 ClientHeight = 2490
 ClientLeft = 2340
 ClientTop = 1935
 ClientWidth = 6195
 ClipControls = 0 'False
 LinkTopic = "Form2"
 MaxButton = 0 False
 MmButton = 0 'False
 ScaleHeight = 1718.642
 ScaleMode = 0 'User
 ScaleWidth = 5817.425
 ShowInTaskbar = 0 'False
 Begin VB.PictureBox picIcon
  AutoSize = -1 True
  ClipControls = 0 False
  Height = 1215
  Left = 120
  Picture = "fmtAbout.frx":0000
  ScaleHeight = 811.195
  ScaleMode = 0 'User
  ScaleWidth = 2401.98
  TabIndex = 1
  Top = 120
  Width = 3480
 End
 Begin VB.CommandButton cmdOK
  Cancel = -1 True
  Caption = "OK"
  Default = -1 True
  Height = 345
  Left = 2520
  TabIndex = 0
  Top = 2040
  'Width = 1260
 End
 Begin VB.Label lblCompany
  Caption = "lblCompany"
  Height = 255
  Left = 3720
  TabIndex = 5
  Top = 1200
  Width = 2295
 End
 Begin VB.Line Line1
  BorderColor = &H00808080&
  BorderStyle = 6 'Inside Solid
  Index = 1
  X1 = 112.686
  X2 = 5746.996
  Y1 = 1325218
  Y2 = 1325218
 End
 Begin VB.Label lblDescription
  Caption = "App Description"
  ForeColor = &H00000000&
  Height = 330
  Left - 120
  TabIndex = 2
  Top = 1560
 Width = 5925
 End
 Begin VB.Label lblTitle
  Caption = "Application Title"
 ForeColor = &H00000000&
 Height = 360
  Left = 3720
 TabIndex = 3
 Top = 240
 Width = 2325
 End
 Begin VB.Line Line1
 BorderColor = &H00FFFFFF&
 BorderWidth = 2
 Index = 0
 X1 = 112.686
 X2 = 5746.996
 Y1 = 1325218
 Y2 = 1325218
 End
 Begin VB.Label lblVersion
 Caption = "Version"
 Height = 225
 Left = 3720
 TabIndex = 4
 Top = 720
 Width = 2325
 End
 End
 Attribute VB_Name = "frmAbout"
 Attribute VB_GlobalNameSpace = False
 Attribute VB_Creatable = False
 Attribute VB_PredeclaredId = True
 Attribute VB_Exposed = False
 Option Explicit
 Private Sub cmdOK_Click()
 Unload Me
 End Sub

 Private Sub Form_Load()
 Me.Caption = "About " & App.Title
 lblVersion.Caption = Version " & App.Major & "." & App.Minor & "." & App.Revision
 lblTitle.Caption = App.Title
 lblDescription.Caption = App.FileDescnption
 lblCompany.Caption = App.CompanyName
 End Sub
```

## Patentansprüche

1. Modifizierte mRNA, die für mindestens ein antigenes virales Peptid oder Polypeptid codiert, **dadurch gekennzeichnet, dass**
der G/C-Gehalt des für das Peptid oder Polypeptid codierenden Bereichs der modifizierten mRNA größer als der G/C-Gehalt des codierenden Bereichs der für das Peptid oder Polypeptid codierenden Wildtyp-mRNA ist und die codierte Aminosäuresequenz gegenüber dem Wildtyp unverändert ist.

2. Modifizierte mRNA nach Anspruch 1, **dadurch gekennzeichnet, dass** der G/C-Gehalt des für das Peptid oder Polypeptid codierenden Bereichs der modifizierten mRNA um mindestens 7% Punkte, bevorzugt um mindestens 15%-Punkte größer als der G/C-Gehalt des codierenden Bereichs der für das Peptid oder Polypeptid codierenden Wildtyp-mRNA ist.

3. Modifizierte mRNA nach einem der Ansprüche 1 bis 2, **dadurch gekennzeichnet, dass** die modifizierte mRNA eine 5'-Cap-Strukmr und/oder einen polyA-Schwanz von mindestens 70 Nucleotiden und/oder eine IRES und/oder eine 5'-Stabilisierungssequenz und/oder eine 3'-Stabilisierungssequenz aufweist.

4. Modifizierte mRNA nach einem der Ansprüche 1 bis 3, **dadurch gekennzeichnet, dass** die modifizierte mRNA mindestens ein Analoges natürlich vorkommender Nucleotide aufweist.

5. Modifizierte mRNA nach Anspruch 4, **dadurch gekennzeichnet, dass** das Analoge aus der Gruppe, bestehend aus Phosphorthioaten, Phosphoramidaten, Peptidnucleotiden, Methylphosphonaten, 7-Deazaguanosin, 5-Methylcytosin und Inosin, ausgewählt ist.

6. Modifizierte mRNA nach einem der Ansprüche 1 bis 5, **dadurch gekennzeichnet, dass** das virale Antigen aus der sekretierten Form eines Oberflächen-Antigens stammt.

7. Modifizierte mRNA nach einem der Anspruche 1 bis 5, **dadurch gekennzeichnet, dass** die mRNA für ein Oberflächen-Antigen von pathogenen viralen Keimen codiert.

8. Modifizierte mRNA nach einem der Ansprüche 1 bis 7, **dadurch gekennzeichnet, dass** die mRNA mit einem kationischen Peptid oder Protein assoziiert oder daran gebunden ist.

9. Modifizierte mRNA nach Anspruch 8, **dadurch gekennzeichnet, dass** das kationische Peptid oder Protein ausgewählt ist aus der Gruppe bestehend aus Protamin, Poly-L-Lysin und Histonen.

10. Modifizierte mRNA nach einem der Ansprüche 1 bis 9, **dadurch gekennzeichnet, dass** das Polypeptid ein Polyepitop von viralen Antigenen ist.

11. Modifizierte mRNA nach einem der Ansprüche 1 bis 10, **dadurch gekennzeichnet, daß** die modifizierte mRNA eine multicistronische mRNA ist.

12. Modifizierte mRNA nach einem der Ansprüche 1 bis 11, **dadurch gekennzeichnet, dass** die mRNA zusätzlich für mindestens ein Cytokin codiert.

13. Modifizierte mRNA nach einem der Ansprüche 1 bis 12, **dadurch gekennzeichnet, dass** die multicistronische RNA mehr als eine IRES-Sequenz aufweist, wobei die IRES-Sequenzen insbesondere ausgewählt sind aus Picornaviren (z.B. FMDV), Pestviren (CFFV), Polioviren (PV), Enzephalo-Myocarditis-Viren (ECMV), Maul-und Klauenseuche-Viren (FMDV), Hepatitis-C-Viren (HCV), Klassisches-Schweinefieber-Viren (CSFV), Murines-Leukoma-Virus (MLV), Simean-Immundefizienz-Viren (SIV) oder Cricket-Paralysis-Viren (CrPV).

14. Pharmazeutische Zusammensetzung, **dadurch gekennzeichnet, dass** sie modifizierte mRNA nach einem der Ansprüche 1 bis 13 in Verbindung mit einem pharmazeutisch verträglichen Träger und/oder Vehikel enthält.

15. Pharmazeutische Zusammensetzung nach Anspruch 14, **dadurch gekennzeichnet, dass** sie mindestens ein die Immunantwort stimulierendes Adjuvans enthält.

16. Pharmazeutische Zusammensetzung nach einem der Ansprüche 14 oder 15, welche weiter mindestens ein Cytokin enthält.

17. Verwendung einer pharmazeutischen Zusammensetzung nach einem der Ansprüche 14 bis 16 oder einer modifizierten mRNA nach einem der Ansprüche 1 bis 13 zur Herstellung eines Impfstoffs zur Impfung gegenvirale Infektionskrankheiten.

18. Verwendung einer pharmazeutischen Zusammensetzung nach Anspruch 17 zur Herstellung eines Impfstoffs zur Impfung gegen AIDS, Hepatitis A, B oder C, Herpes, Herpes Zoster, Röteln, Dengue, hämorrhagische Infektionserkrankungen Gelbfieber, und Grippe.

## Claims

1. Modified mRNA coding for at least one antigenic viral peptide or polypeptide, **characterised in that** the G/C content of the region of the modified mRNA coding for the peptide or polypeptide is increased compared to the G/C content of the coding region of the wild type mRNA coding for the peptide or polypeptide, and the encoded amino acid sequence is unchanged as compared to the wild type.

2. Modified mRNA according to claim 1, **characterised in that** the G/C content of the region of the modified mRNA coding for the peptide or polypeptide is increased by at least 7 % points, preferably at least 15 % points, compared to the G/C content of the coding region of the wild type mRNA coding for the peptide or polypeptide.

3. Modified mRNA according to one of claims 1 to 2, **characterised in that** the modified mRNA comprises a 5' cap structure and/or a poly-A tail of at least 70 nucleotides and/or an IRES and/or a 5' stabilisation sequence and/or a 3' stabilisation sequence.

4. Modified mRNA according to one of claims 1 to 3, **characterised in that** the modified mRNA comprises at least one analogue of naturally occurring nucleotides.

5. Modified mRNA according to claim 4, **characterised in that** the analogue is selected from the group consisting of phosphorus thioates, phosphorus amidates, peptide nucleotides, methylphosphonates, 7-deazaguanosine, 5-methylcytosine and inosine.

6. Modified mRNA according to one of claims 1 to 5, **characterised in that** the viral antigen derives from the secreted form of a surface antigen.

7. Modified mRNA according to one of claims 1 to 5, **characterised in that** the mRNA codes for a surface antigen of pathogenic viral germs.

8. Modified mRNA according to one of claims 1 to 7, **characterised in that** the mRNA is associated with a cationic peptide or protein or is bound thereto.

9. Modified mRNA according to claim 8, **characterised in that** the cationic peptide or protein is selected from the group consisting of protamine, poly-L lysine, and histones.

10. Modified mRNA according to one of claims 1 to 9, **characterised in that** the polypeptide is a polyepitope of viral antigens.

11. Modified mRNA according to one of claims 1 to 10, **characterised in that** the modified mRNA is a multicistronic mRNA.

12. Modified mRNA according to one of claims 1 to 11, **characterised in that** the mRNA in addition codes for at least one cytokine.

13. Modified mRNA according to one of claims 1 to 12, **characterised in that** the mRNA comprises one or more IRES sequence(s), wherein the IRES sequences are in particular selected from picorna viruses (e.g. FMDV), plague viruses (CFFV), polio viruses (PV), encephalo myocarditis viruses (ECMV), foot-and-mouth disease viruses (FMDV), hepatitis C viruses (HCV), classic swine fever viruses (CSFV), murine leukemia virus (MLV), simian immunodefiency viruses (SIV), or cricket paralysis viruses (CrPV).

14. Pharmaceutical composition **characterized in that** it contains a modified mRNA according to one of claims 1 to 13 in combination with a pharmaceutically acceptable carrier and/or vehicle.

15. Pharmaceutical composition according to claim 14, **characterised in that** the pharmaceutical composition contains at least one the immune response stimulating adjuvant.

16. Pharmaceutical composition according to one of claims 14 or 15, which in addition contains at least one cytokine.

17. Use of a pharmaceutical composition according to one of claims 14 to 16 or a modified mRNA according to one of claims 1 to 13 for the preparation of a vaccine for inoculation against viral infectious diseases.

18. Use of a pharmaceutical composition according to claim 17 for the preparation of a vaccine for inoculation against AIDS, hepatitis A, B or C, herpes, herpes zoster, rubella, dengue, haemorrhagic infectious diseases, yellow fever and influenza.

## Revendications

1. ARNm modifié qui code pour au moins un peptide ou polypeptide antigène viral, **caractérisé en ce que** la teneur en G/C de la région de l'ARNm modifié codant pour le peptide ou polypeptide est augmentée par rapport à la teneur en G/C de la région codante de l'ARNm de type sauvage codant pour le peptide ou polypeptide, et **en ce que** la séquence d'acide aminés codée est inchangée par rapport au type sauvage.

2. ARNm modifié selon la revendication 1, **caractérisé en ce que** la teneur en G/C de la région de l'ARNm modifié codant pour le peptide ou polypeptide est augmentée d'au moins 7 points en pourcentage, préféré d'au moins 15 points en pourcentage, par rapport à la teneur en G/C de la région codante de l'ARNm de type sauvage codant pour le peptide ou polypeptide.

3. ARNm modifié selon l'une des revendications 1 à 2, **caractérisé en ce que** l'ARNm modifié comporte une structure de coiffe 5' et/ou une queue poly-A d'au moins 70 nucleotides et/ou une séquence IRES et/ou une séquence de stabilisation 5' et/ou une séquence de stabilisation 3'.

4. ARNm modifié selon l'une des revendications 1 à 3, **caractérisé en ce que** l'ARNm modifié comporte au moins un analogue de nucléotides se rencontrant dans la nature.

5. ARNm modifié selon la revendication 4, **caractérisé en ce que** l'analogue est choisi dans le groupe consistant en des phosphothioates, des phosphoamidates, des nucléotides peptidiques, des phosphonates de méthyle, la 7-déazaguanosine, la 5-méthylcytosine et l'inosine.

6. ARNm modifié selon l'une des revendications 1 à 5, **caractérisé en ce que** l'antigène viral provient d'une forme sécrétée d'un antigène de surface.

7. ARNm modifié selon l'une des revendications 1 à 5, **caractérisé en ce que** l'ARNm code pour un antigène de surface des germes virales pathogènes.

8. ARNm modifié selon l'une des revendications 1 à 7, **caractérisé en ce que** l'ARNm est associée à un peptide ou une protéine cationique, ou est lieé à ces derniers.

9. ARNm modifié selon la revendication 8, **caractérisé en ce que** le peptide ou protéine cationique et choisi dans le groupe consistant en protamine, poly-L-lysine, ou les histones.

10. ARNm modifié selon l'une des revendications 1 à 9, **caractérisé en ce que** le polypeptide est un polyépitope d'antigènes viral.

11. ARNm modifié selon l'une des revendications 1 à 10, **caractérisé en ce que** l'ARNm modifié est un ARNm multicistronique.

12. ARNm modifié selon l'une des revendications 1 à 11, **caractérisé en ce que** l'ARNm code en outre pour au moins une cytokine.

13. ARNm modifié selon l'une des revendications 1 à 12, **caractérisé en ce que** l'ARNm comporte un ou plusieurs sequences d'IRES, qui en particulier sont choisi dans le groupe consistant en picornavirus (par exemple FMDV), des virus de la peste (CFFV), des virus de la polio (PV), des virus d'encéphalomyorcardite (ECMV), des virus de fièvre aphteuse (FMDV), des virus de l'hépatite C (HCV), des virus de la peste porcine classique (CSFV), des virus de la leucémie murine (MLV), des virus de l'immunodéficience simienne (SIV) ou des virus de paralysie du grillon (CrPV).

14. Composition pharmaceutique, **caractérisée en ce qu'**elle contient de l'ARNm modifié selon une des revendications 1 à 13 combiné à un support et/ou à un véhicule pharmacologiquement compatible.

15. Composition pharmaceutique selon la revendication 14, **caractérisée en ce qu'**elle contient au moins un adjuvant stimulant la réponse immunitaire.

16. Composition pharmaceutique selon l'une des revendications 14 ou 15, qui contient en outre au moins une cytokine.

17. Utilisation d'une composition pharmaceutique selon l'une des revendications 14 à 16 ou d'un ARNm modifié selon l'une des revendications 1 à 13 pour la préparation d'un vaccin destiné à la vaccination contre les maladies infectieuses virales.

18. Utilisation d'une composition pharmaceutique selon la revendication 17 pour la préparation d'un vaccin destiné à la vaccination contre le SIDA, l'hépatite A, B ou C, l'herpès, l'herpès zoster, la rubéole, la dengue, les maladies infectieuses hémorragiques, la fièvre jaune ou la grippe.
